# FASCICULE DE BREVET EUROPEEN

(11) **EP 3 863 559 B1**
(45) Date de publication et mention de la délivrance du brevet: **07.06.2023**
(21) Numéro de dépôt: 19784087.9
(22) Date de dépôt: 15.10.2019
(51) Int. Cl.: A61C 13/00, A61C 1/08

(54) **MÉTHODE DE CONCEPTION D'UN ÉLÉMENT PROTHÉTIQUE**
METHODE ZUM ENTWERFEN EINES PROTHETISCHEN ELEMENTS
METHOD FOR DESIGNING A PROSTHETIC ELEMENT

(30) Priorité: 15.10.2018 EP 18200412
(43) Date de publication de la demande: 18.08.2021
(73) Titulaire: Dental Design, 1180 Uccle (BE)
(72) Inventeur: CHELALA, Pierre, 1410 Waterloo (BE)
(74) Mandataire: Gevers Patents
(86) Numéro de dépôt international: PCT/EP2019/077935
(87) Numéro de publication internationale: WO 2020/078989

(56) Documents cités:
- WO-A1-2018/007935
- WO-A1-2018/154485
- US-A1- 2009 148 816
- US-A1- 2012 143 364
- US-A1- 2013 230 827
- US-A1- 2018 263 726

## Description

### Domaine technique

L'invention concerne une méthode de conception d'un élément prothétique.

### Art antérieur

De nos jours, lorsqu'une restauration dentaire est à effectuer sur une dent d'un patient via la pose d'un élément prothétique, il est usuel pour un dentiste de procéder à une préparation de la dent comprenant par exemple les étapes suivantes : taille de la dent, suivie d'une prise d'empreinte de la dent taillée. Cette empreinte est envoyée dans un laboratoire pour conception de l'élément prothétique, celui-ci étant ensuite essayé, éventuellement adapté, et enfin posé sur la dent taillée du patient.

Malgré l'avènement de méthodes informatiques permettant la prise de cette empreinte numériquement, l'ensemble de ce processus de restauration dentaire est long et nécessite un certain nombre de rendez-vous du patient chez son dentiste.

En outre, la taille de la dent par le dentiste, les matériaux d'empreintes et le flux de fabrication au laboratoire sont des sources régulières d'erreurs techniques mettant en péril l'effet thérapeutique attendu par une restauration dentaire. En particulier, il est complexe pour le dentiste de tenir compte de la structure globale des tissus durs et mous associés à la dentition du patient, de sorte que la taille n'est que rarement parfaitement adaptée à la fois au patient et aux contraintes techniques du prothésiste produisant l'élément prothétique.

Le document WO2018154485 décrit une méthode de assistée par ordinateur permettant de générer des instructions chirurgicales pour la réduction volumétrique sur une dent et produire un élément prothétique correspondant. Dans les étapes préparatoires un algorithme permet de comparer une image radiographie sur une image 3D du site chirurgical.

### Résumé de l'invention

Un objet de l'invention est de fournir une méthode de conception d'un élément prothétique davantage adapté aux besoins d'un patient, permettant une fabrication dudit élément prothétique et un traitement du patient qui soient plus rapides, moins chers et plus fiables.

À cet effet, la présente invention propose une méthode de conception d'un élément prothétique comprenant les étapes suivantes, exécutées dans cet ordre :
(i)
   - fournir un premier fichier informatique comprenant :
      - une représentation tridimensionnelle intra-orale d'une dentition comprenant au moins une dent à restaurer au moyen de l'élément prothétique ;
      - une image radiographique de la dentition ;
   - identifier des axes de référence communs sur la représentation tridimensionnelle intra-orale et sur l'image radiographique ;
   - comparer la représentation tridimensionnelle intra-orale avec l'image radiographique, cette sous-étape de comparaison comprenant une superposition des axes de référence communs ;
(ii) déterminer une représentation tridimensionnelle d'un extrados de l'élément prothétique sur base du premier fichier informatique ;
(iii) déterminer des paramètres techniques comprenant :
   - un protocole dentaire, et/ou
   - un type de préparation dentaire, et/ou
   - des contraintes techniques,
   sur base du premier fichier informatique, au moins un des paramètres techniques étant déterminé sur base de la sous-étape de comparaison de l'étape (i) ;
(iv) générer un deuxième fichier informatique comprenant une représentation tridimensionnelle d'une réduction volumétrique de la au moins une dent sur base des paramètres techniques ;
(v) valider et/ou modifier le deuxième fichier informatique ;
(vi) obtenir une représentation tridimensionnelle d'un intrados de l'élément prothétique sur base du deuxième fichier informatique validé et/ou modifié ;
(vii) générer un troisième fichier informatique comprenant des informations relatives aux représentations tridimensionnelles des extrados et intrados de l'élément prothétique ;
(vii') générer un quatrième fichier informatique sur base dudit deuxième fichier informatique validé et/ou modifié, ledit quatrième fichier informatique comprenant des instructions d'usinage de la au moins une dent correspondant à la représentation tridimensionnelle de la réduction volumétrique de la au moins une dent;
(viii) produire l'élément prothétique sur base du troisième fichier informatique.

La méthode de conception d'un élément prothétique selon l'invention permet de mettre en oeuvre un processus de restauration dentaire particulièrement efficace, peu coûteux, et rapide, en fournissant un élément prothétique parfaitement adapté aux besoins d'un patient.

En effet, cette méthode concerne principalement des étapes numériques effectuées préalablement à la restauration dentaire. En particulier, les étapes (ii) à (vii) sont au coeur de la présente invention, et sont effectuées au moyen d'outils informatiques, en arrière plan de la restauration dentaire. Elles permettent d'obtenir un modèle numérique d'élément prothétique susceptible d'être parfaitement adapté aux besoins d'un patient sans qu'aucune taille de la au moins une dent soit nécessaire au préalable. L'étape (ii) concerne essentiellement l'esthétique externe de l'élément prothétique dont un extrados est déterminé en correspondance avec le premier fichier informatique, et donc de façon globale, avec la dentition du patient et la structure globale des tissus mous et durs associés à cette dentition. Les étapes (iii) à (vii) permettent, entre autre, de déterminer un intrados de l'élément prothétique. Cet intrados doit s'adapter de façon parfaite à une taille de la au moins une dent. Néanmoins, dans le cas de la présente invention, cette taille n'a préférentiellement pas été effectuée de façon concrète et réelle au préalable, mais bien de préférence virtuellement, aux étapes (iv) et (v), définie sous la forme d'une réduction volumétrique de la au moins une dent basée sur les informations du premier fichier informatique fourni à l'étape (i) et sur les paramètres déduits de ces informations à l'étape (iii), préférentiellement par un informaticien spécialisé et/ou un prothésiste expérimenté. Ainsi, l'intrados est déterminé a posteriori essentiellement sur base de cette réduction volumétrique. Le deuxième fichier informatique modifié et/ou validé comprenant la représentation tridimensionnelle de la réduction volumétrique contient donc toutes les informations nécessaires à réalisation de cette réduction volumétrique, celle-ci permettant de tailler ultérieurement la au moins une dent de façon à ce que sa surface externe taillée corresponde exactement à l'intrados de l'élément prothétique. Ainsi, et très avantageusement, il est possible de définir numériquement et de produire l'élément prothétique grâce à la méthode de conception selon la présente invention, et, seulement après, de tailler la au moins une dent sur base de la définition de la réduction volumétrique de la au moins une dent contenue dans le deuxième fichier informatique après validation et/ou modification. L'élément prothétique produit est alors plus adapté aux besoins d'un patient, en particulier, à sa dentition et aux tissus mous et durs qui y sont associés, ainsi qu'aux contraintes techniques, par exemple, des tolérances, de fabrication d'un élément prothétique, qu'un autre élément prothétique qui serait produit sur base d'une empreinte de la au moins une dent taillée de façon plus approximative par le dentiste.

Dans ce contexte, la réduction volumétrique (définissant de préférence une taille) de la au moins une dent est donc calculée avant l'intrados de l'élément prothétique, bien que par la suite, dans la pratique, ce soit l'élément prothétique qui soit produit avant (ou en même temps) que la réduction volumétrique.

Dans la suite proche du résumé, il est commenté précisément sur les étapes de méthode de conception selon l'invention. Il est ensuite présenté différent avantages de cette méthode, notamment dans la mise en oeuvre d'une restauration dentaire.

L'étape (i) permet de façon avantageuse de fournir un grand nombre d'informations sur la dentition et les tissus mous et durs associés à cette dentition à un prothésiste dentaire et/ou un CAD designer pour définir plus précisément les besoins du patient, et donc un élément prothétique davantage adapté par les étapes (ii) à (vii). Il est proposé de fournir une (ou plusieurs) représentation tridimensionnelle intra-orale de la dentition de préférence non taillée. Cette sous-étape peut être effectuée en moins de trente secondes au moyen d'un scanner intra-oral connu d'un homme du métier. L'image radiographique peut être fournie par tout appareil à rayons X connu d'un homme du métier. Il s'agit préférentiellement d'une image bidimensionnelle. Une telle image est plus facile et moins coûteuse à produire tout en donnant suffisamment d'informations pertinentes pour la sous-étape de comparaison. Une image radiographique tridimensionnelle (par exemple, produite par une technique d'imagerie volumétrique par faisceau conique, ou CBCT) n'est cependant pas exclus du cadre de l'invention. Les images fournies par cette étape (i) permettent de définir très précisément un type de préparation et/ou des contraintes techniques à l'étape (iii), notamment grâce à la sous-étape de comparaison. En effet, s'il est contre intuitif de superposer des données tridimensionnelles et bidimensionnelles (pour une image radiographique bidimensionnelle) cette superposition suffit à identifier, par exemple, la chambre pulpaire et le système nerveux associé à la au moins une dent afin d'en tenir pleinement compte dans la détermination des paramètres techniques à l'étape (iii). En particulier, une telle image radiographique est utile dans le cas d'une restauration dentaire nécessitant une retaille d'une certaine dent, par exemple, dans le cas d'un retrait d'un ancien élément prothétique. Ainsi, il est plus aisé d'identifier la certaine dent, ainsi que les corps mous et durs qui lui sont associés.

L'étape (i) comprend également, une identification des axes de référence communs sur la représentation tridimensionnelle intra-orale et sur l'image radiographique, et une superposition des axes de référence communs lors de la sous-étape de comparaison. Ces axes jouent un rôle primordial dans le travail du prothésiste dentaire et/ou du dentiste car ils permettent de définir un repère de travail numérique adéquat sur la au moins une dent. En particulier, ils comprennent de préférence un axe d'insertion de la au moins une dent. Il est donc crucial que ces axes soient déterminés de façon très précise. Ladite représentation tridimensionnelle intra-orale est suffisante pour déterminer de tels axes, mais ladite image radiographique permet de légère adaptation de ces axes de façon à tenir compte d'éléments non visibles sur l'image radiographique tels que la chambre pulpaire et/ou le système nerveux associés à la au moins une dent. Ainsi la comparaison des images par superposition des axes livre une excellente base à la définition d'un repère de travail numérique idéal pour les étapes ultérieures de la méthode, tout en permettant de détecter des contraintes techniques qui ne seraient pas directement apparentes sur une seule représentation tridimensionnelle intra-orale. En général, l'étape (i) est très avantageuse pour planifier de façon à la fois intelligente et précise une réduction volumétrique (et donc une taille) de la au moins une dent.

Optionnellement, l'étape (i) comprend également une étape de fourniture d'une image capturée par UV et de sa superposition et/ou comparaison avec les autres données du premier fichier informatique pour détecter la présence de carie, de cavité cariées et/ou d'amalgames.

Les étapes (ii) à (vii) de la méthode de conception selon la présente invention constituent des étapes de planification et préparation intelligentes préalables au bon déroulement d'une restauration dentaire. Différents logiciels connus d'un homme du métier permettent de simuler et/ou de manipuler une image tridimensionnelle d'une dentition restaurée. Il est possible d'obtenir un design d'un extrados probable d'un élément prothétique pour restaurer la au moins une dent à partir d'une telle simulation. Cette obtention de design est néanmoins superficielle car elle n'indique ni comment procéder à une restauration dentaire, ni comment produire un élément prothétique. En outre, de par même son but, ce design ne tient pas compte de nombreux protocoles et tolérances de fabrication d'un élément prothétique adapté aux besoins d'un patient. C'est pourquoi la méthode de conception selon l'invention propose d'étudier le premier fichier informatique à l'étape (i), de façon à en déduire les paramètres techniques de l'étape (iii). Ces paramètres techniques comprennent de préférence au moins un parmi : des informations sur la dentition, les racines dentaires, les gencives, le tissu parodontal, un index gingival, des dents qui sont adjacentes et/ou antagonistes à la au moins une dent, des informations d'alignement et d'encombrement des dents, les zones buccales d'accès, la nature d'un matériau de conception de l'élément prothétique ainsi que les tolérances associées à ce matériau, et en particulier, l'épaisseur minimale que doivent avoir les parois de l'élément prothétique, et/ou des paramètres propres à un centre de production de l'élément prothétique. Ces éléments font de préférence partie intégrante d'un protocole dentaire déterminé directement et algorithmiquement à partir de l'étape (i), et en particulier donc sur base du premier fichier informatique. Préférentiellement, l'étape (i) permet de déterminer un type de préparation dentaire (comprenant par exemple, le type de préparation dentaire à réaliser, la au moins une dent à restaurer, le type d'élément prothétique et l'épaisseur minimale qu'il doit avoir, etc.) et de préférence aussi un axe d'insertion de la au moins une dent parmi les axes de référence et/ou des axes de taille numérique parmi ces axes de référence, ces données étant fournies comme entrées d'un algorithme permettant avantageusement de générer algorithmiquement un protocole dentaire sur cette base. Ceci est discuté avantageusement plus loin dans ce résumé.

De cette façon, toute l'expérience d'un prothésiste est mise au service d'une conception d'un élément prothétique, par l'établissement automatique à l'étape (iv) d'un plan de réduction volumétrique de la au moins une dent prenant en compte l'ensemble de ces paramètres. Cet établissement automatique est préférentiellement réalisé sur base d'un programme d'ordinateur conçu spécialement avantageusement dans le cadre de la présente invention, prenant de préférence comme entrée un protocole dentaire. Cet étape (iv) donne lieu à la génération du deuxième fichier informatique. L'étape (v) de valider et/ou modifier son contenu, et plus spécifiquement la représentation tridimensionnelle de la réduction volumétrique de la au moins une dent. Différents paramètres sont ainsi préférentiellement contrôler par une personne habilitée, par exemple, un prothésiste dentaire.

Ces étapes (iv) et (v) sont très avantageuses car elles permettent de bénéficier d'une aide pour déterminer des paramètres à prendre en compte pour définir virtuellement un élément prothétique tout en permettant une liberté de validation et/ou de modification de ce deuxième fichier informatique obtenu. En particulier, le deuxième fichier informatique est modifiable et n'est pas le résultat d'un logiciel fermé fournissant un design automatique d'élément prothétique non exploitable.

Préférentiellement, le deuxième fichier informatique est un fichier CAD modifiable, de préférence de format STL, définissant une taille virtuelle de la au moins une dent sur base des étapes (i) à (iii), de sorte qu'il constitue un fichier qui peut être délivré de l'étape (iv), mais également exploité et modifié à l'étape (v) et dans les étapes ultérieures. L'étape (v) comprend préférentiellement une validation et/ou une modification de chacun parmi des paramètres géométriques relatifs à la représentation tridimensionnelle de la réduction volumétrique de la au moins une dent. De préférence, ces paramètres géométriques sont parmi :
- des lignes de marges périphériques,
- des plans parallèles bordant des côtés latéraux de la au moins une dent,
- un contour gingival de la au moins une dent,
- des axes de taille de la au moins une dent,
- des zones de réduction volumétrique de la au moins une dent,
- des faces de réduction volumétrique de la au moins une dent,
- des grandeurs de réduction volumétrique pour chacune des zone de réduction volumétrique de la au moins une dent.
Tout ces paramètres sont à prendre en compte dans l'établissement d'un élément prothétique. Plus préférentiellement, chacun des paramètres géométriques est modifiable dans un ensemble de valeurs admissibles préalablement défini par au moins un des paramètres techniques déterminé à l'étape (iii), de préférence, par le protocole dentaire. De cette façon, un technicien peu expérimenté ou distrait ne commettrait pas d'erreurs dans la conception de l'élément prothétique.

Le deuxième fichier informatique ainsi validé et/ou modifié comprend par conséquent une représentation tridimensionnelle de la réduction volumétrique de la au moins une dent, et donc peut servir de base à l'obtention en l'étape (vi) d'une représentation tridimensionnelle de l'intrados de l'élément prothétique, celui-ci étant complémentaire à une surface externe de la au moins une dent réduite de façon volumétrique. Le deuxième fichier informatique et l'ensemble des représentations ainsi définies de l'intrados et de l'extrados de l'élément prothétique constituent préférentiellement des fichiers informatiques CAD connus d'un homme du métier mais avantageusement exploitable selon l'invention. Le design de l'élément prothétique étant défini, il est alors généré un troisième fichier informatique, de préférence de type CAM, comprenant des informations relatives à ces représentations tridimensionnelles d'intrados et d'extrados définissant le design de l'élément prothétique. Ces informations servent de base à la production de l'élément prothétique à l'étape (viii) sans perdre la possibilité d'exploiter les informations du deuxième fichier informatique pour guider un praticien dans la taille de la au moins une dent afin de correspondre avec la réduction volumétrique précédemment définie.

Ce troisième fichier informatique se distingue du deuxième fichier informatique en ce qu'il comprend des informations techniques préférentiellement directement exploitables lors d'une production de l'élément prothétique, notamment par une machine de production, alors que le deuxième fichier informatique comprend préférentiellement des données de représentation tridimensionnelles. L'obtention du troisième fichier informatique à partir du deuxième ne se fait pas par des logiciels existants mais à partir d'un programme d'ordinateur selon l'invention pour tenir compte à nouveau des paramètres techniques, et préférentiellement très spécifiquement pour tenir compte de protocoles de conception de l'éléments prothétique et de protocoles dentaires d'usinage dentaire. De préférence, le troisième fichier informatique est également un fichier CAM, optionnellement de stéréolithographie.

De façon très préférée, les informations du troisième fichier informatique comprennent des instructions d'usinage d'un matériau, et l'étape (viii) comprend une sous-étape d'usinage du matériau sur base des instructions d'usinage. De cette façon, une machine d'usinage apte à lire les instructions peut usiner un matériau pour lui donner une forme creuse comprenant une face interne analogue à la représentation de l'intrados et une forme externe analogue à la représentation de l'extrados. Le matériau comprend préférentiellement au moins un parmi de la céramique, du zircone, de la céramique hybride, du composite, de la résine, et ce de façon à concevoir un élément prothétique solide et biocompatible, permettant au patient de bénéficier d'une restauration dentaire saine, durable et stable. Préférentiellement, le matériau consiste en de la biocéramique adaptée à un usinage.

De préférence, l'étape (ii) de la méthode comprend les sous-étapes suivantes :
(ii.1) choisir une représentation tridimensionnelle modèle d'une dentition modèle dans une base de données sur base du premier fichier informatique ;
(ii.2) choisir une zone de la représentation tridimensionnelle modèle correspondant à une zone de la représentation tridimensionnelle intra-orale correspondant à la au moins une dent ;
(ii.3) valider et/ou modifier la zone de la représentation tridimensionnelle modèle sur base du premier fichier informatique ;
(ii.4) définir la représentation tridimensionnelle de l'extrados de l'élément prothétique à partir de la zone validée et/ou modifiée de la représentation tridimensionnelle modèle.

Cette réalisation préférée de la méthode comprend de façon essentielle un choix de représentation tridimensionnelle d'une dentition sans défaut simulant la représentation tridimensionnelle de la dentition comprenant la au moins une dent parmi une base de donnée (ou bibliothèque) de telles représentations. Ainsi, il est possible de déterminer une forme finale de dentition restaurée adaptée aux besoins d'un patient, et d'en tirer la forme d'une enveloppe externe à donner à l'élément prothétique pour optimiser l'esthétique de cette nouvelle forme finale de dentition. Cette enveloppe externe consiste de façon basique en l'extrados de l'élément prothétique. Vu qu'il se peut qu'aucune représentation tridimensionnelle modèle ne corresponde exactement à l'extrados souhaité, il est prévu une étape de validation et/ou modification analogue à celle de l'étape (v) détaillée ci-dessus pour permettre à un prothésiste dentaire et/ou un CAD designer d'effectuer des retouches sur cette zone. Celles-ci peuvent par exemple concerner un axe d'insertion, un axe et/ou une hauteur de contact de la au moins une dent avec des dents antagonistes et/ou adjacentes, ou encore un alignement dentaire. Il est de façon préférée tenu compte d'un index gingival pour définir la représentation tridimensionnelle de l'extrados.

De façon générale, la méthode de conception selon l'invention permet une inversion d'une étape globale de préparation de la au moins une dent avec une conception de l'élément prothétique, et ce dans un processus global de restauration dentaire qui se distingue alors très avantageusement de l'art antérieur, en ce qu'il est considérablement plus efficace et rapide. En particulier, ce processus de restauration dentaire propose une préparation de la au moins une dent comprenant une taille de la au moins une dent adaptée à l'élément prothétique et non l'inverse ! Un exemple d'un mode de réalisation très préféré d'un tel processus de restauration dentaire à partir de la méthode de conception selon l'invention est décrit dans la description détaillée ci-après. Plus spécifiquement, la longue séquence de rendez-vous entre le patient et le dentiste peut de façon avantageuse être remplacée par un rendez-vous unique au cours duquel, d'une part, la au moins une dent est taillée par un dentiste en fonction d'informations du deuxième fichier informatique, et, d'autre part, l'élément prothétique produit est posé sur la au moins une dent taillée. Par exemple, dans le cas où l'élément prothétique est une couronne d'une dent, cette restauration dentaire peut être effectuée lors d'un seul rendez-vous d'une durée inférieure à trente minutes. La taille de la au moins une dent sur base des informations du deuxième fichier informatique est davantage exacte et précise, car elle est basée sur des informations rigoureuses, étudiées et analysées par un ou plusieurs spécialistes, et ce sur base de méthode numériques fiables, loin des conditions stressantes de travail du dentiste lors d'une opération de chirurgie dentaire. La comptabilité entre la taille de la au moins une dent, et l'élément prothétique, d'une part, et la structure globale des tissus et de la dentition, d'autre part, est ainsi assurée et parfaitement prédictible. Aucune prise d'empreinte ne doit être effectuée après la taille de la au moins une dent car il est acquis que l'élément prothétique sera adapté à cette taille avec une grande précision, à la condition où cette préparation est effectuée sur base d'informations du deuxième fichier informatique.

Avantageusement, la méthode de conception selon l'invention permet donc d'éviter de nombreux aller-retour de l'élément prothétique entre un cabinet dentaire et un laboratoire où il est conçu, de tels aller-retour étant parfois nécessaire selon l'état de la technique pour des adaptations de l'élément prothétique, lorsqu'un essai de pose de celui-ci sur la dent du patient n'est pas concluant. En outre, et avantageusement, la méthode de conception selon l'invention permet une large diminution de souffrances d'un patient devant subir une restauration dentaire vu que l'élément prothétique peut être placé parfaitement en une seule séance.

Avantageusement, la méthode de conception selon l'invention permet de produire des éléments prothétiques possédant les avantages susmentionnés à bas coût pour le patient car des étapes d'empreintes dentaires et/ou de modifications éventuelles de l'élément prothétique produit sur base d'une taille de la au moins une dent ne sont plus effectuées. Il est estimé une réduction avantageuse de ce coût à un tiers d'un montant de conception du même élément prothétique au moyen de techniques selon l'art antérieur. Pour une couronne, ce coût est estimé à 300 €. Le coût global d'une restauration dentaire imputable à la production d'un élément prothétique est donc très fortement réduit, rendant ainsi les restaurations dentaires davantage démocratiques pour une clientèle défavorisée.

La méthode de conception selon l'invention, et le processus de restauration dentaire sous-jacent répondent avantageusement à un besoin sociétal grandissant compte tenu du vieillissement accéléré de certaines populations, notamment en Europe, aux USA, au Canada ou en Asie. La méthode de conception selon l'invention permet de produire rapidement et efficacement des éléments prothétiques d'une grande qualité et parfaitement adaptés aux besoins de chaque patient, répondant ainsi à une demande croissante de restauration dentaire tout en compensant pleinement une pénurie dans le personnel soignant, notamment prothésistes ou chirurgiens-dentistes.

Avantageusement, la méthode de conception selon l'invention permet de limiter l'impact écologique et environnemental dû à une délocalisation d'une production d'éléments prothétiques. En effet, il est possible d'exécuter la méthode de conception selon l'invention de façon locale, au sein d'un laboratoire ou d'un cabinet dentaire, pour produire des éléments prothétiques à bas coût, sans avoir recours à une main d'oeuvre étrangère et bon marché. En outre, la méthode de conception étant de façon principale numérique, il peut être joint une étape de vérification d'un respect des normes de sécurité et de traçabilité propres à un élément prothétique.

Il est maintenant présenté un avantageux mode de réalisation préféré de l'invention selon lequel l'étape (iii) comprend les sous-étapes suivantes :
(iii.1) déterminer un type de préparation dentaire sur base dudit premier fichier informatique ;
(iii.2) générer algorithmiquement un protocole dentaire sur base du type de préparation dentaire déterminé à l'étape (iii.1), ledit protocole dentaire consistant en une collection de données numériques pour paramétrer géométriquement ladite représentation tridimensionnelle de la réduction volumétrique ;
(iii.3) valider et/ou modifier ledit protocole dentaire sur base de la sous-étape de comparaison de l'étape (i).

Ce mode de réalisation préféré de l'étape (iii) avait été abordé plus haut. Il est sous-jacent à un programme d'ordinateur permettant de convertir des données sur le type de préparation dentaire en un protocole dentaire étudié expérimentalement de façon à mettre toute l'expérience de l'inventeur au service de la conception d'éléments prothétiques. Ce mode de réalisation facilite avantageusement la réalisation de l'étape (iii) en minimisant les risques pour un prothésiste dentaire peu expérimenté de se tromper. Un temps très précieux dans l'exécution de la méthode selon l'invention est ainsi gagné. De façon plus précise et plus préférée, l'étape (iii.1) comprend les sous-étapes suivantes :
- visualiser ladite représentation tridimensionnelle intra-orale d'une dentition ;
- segmenter ladite représentation tridimensionnelle intra-orale d'une dentition de façon à obtenir une représentation tridimensionnelle isolée de la au moins une dent ;
- générer algorithmiquement des faces vestibulaire, linguale, mésiale, distale et occlusale de la au moins une dent par identification d'un point de chacune de ces faces au niveau de la représentation tridimensionnelle isolée de la au moins une dent ;
- modifier et/ou valider des frontières desdites faces vestibulaire, linguale, mésiale, distale et occlusale de la au moins une dent (91) par des ajouts, des déplacements et/ou des retraits de points de ces faces sur la représentation tridimensionnelle isolée de la au moins une dent ;
- identifier un repère d'au moins un desdits axes de référence sur base de la sous-étape de comparaison de l'étape (i), le repère comprenant un axe d'insertion de la au moins une dent.

Le type de préparation dentaire est ainsi déterminé au regard de manipulations numériques de la représentation tridimensionnelle intra-orale permettant de reconnaître les différentes faces de la dent et de figer un repère de travail sur cette représentation tridimensionnelle. Le praticien peut se rendre compte par ces manipulations numériques et grâce à la sous-étape de comparaison de l'étape (i) (ayant permis une avantageuse détection d'éléments de la dentition, des tissus parodontaux, des tissus mous et durs, des nerfs, etc.) du type de préparation dentaire (comprenant la réduction volumétrique) qu'il convient de prévoir sur la au moins une dent, et du repère nécessaire pour procéder à cette préparation dentaire. Ces modes de réalisation permettent un effet synergique des avantages des étapes (i) et (iii).

De préférence, suite à la sous-étape de segmentation, il est algorithmiquement tenu compte du parodonte interproximale dans le protocole dentaire (par continuation selon une courbe de Bézier). Ceci permet, dans l'étape (iv), de tenir compte d'espaces à combler consécutifs à cette sous-étape de segmentation.

Le protocole dentaire se présente de préférence sous la forme d'un tableau de nombres réels correspondant aux données numériques qui permettent une paramétrisation géométrique de la réduction volumétrique selon des relations avantageuses (indirectes) faisant partie intégrante de l'invention. De préférence, les données numériques comprennent :
- pour chaque face parmi les faces vestibulaire, linguale, mésiale et distale de ladite au moins une dent :
   - un rayon ;
   - une hauteur ;
   correspondant à une paramétrisation d'une section transverse d'un congé elliptique (optionnellement circulaire) de la face selon un arc d'ellipse (optionnellement de cercle) de demi grand-axe correspondant audit rayon mesuré essentiellement perpendiculairement audit axe d'insertion et de demi petit-axe correspondant à ladite hauteur mesurée de façon essentiellement parallèle audit axe d'insertion ;
- pour chaque face parmi les faces vestibulaire, linguale, mésiale et distale de ladite au moins une dent :
   une première donnée de décalage correspondant à un déplacement de chaque point de la face sur la représentation tridimensionnelle isolée de la au moins une dent vers l'axe d'insertion ;
- pour la face occlusale de ladite au moins une dent :
   une première donnée de décalage correspondant à un déplacement de chaque point de la face occlusale sur la représentation tridimensionnelle isolée de la au moins une dent le long de l'axe d'insertion ;
- deux pourcentages définissant deux zones consistant en des zones marginale et médiane de ladite au moins une dent sur la représentation tridimensionnelles isolée de la au moins une dent, chaque pourcentage correspondant au rapport entre une hauteur d'une zone et une hauteur des deux zones (celle-ci correspondant de préférence à une hauteur de couronne dentaire visible, la hauteur de la racine dentaire étant donc non comprise), ces hauteurs étant mesurées essentiellement parallèlement à l'axe d'insertion ;
- pour chacune desdites zones :
   - un angle d'orientation de réduction volumétrique mesuré par rapport à l'axe d'insertion ;
   - une hauteur minimale.
L'axe d'insertion est connu d'un homme du métier. Il est dirigé de la zone marginale vers une face occlusale de la au moins une dent, ou, autrement dit, de la racine vers la face occlusale de la au moins une dent, le long de la au moins une dent. Les données de décalage correspondent de façon préférée à un déplacement vers l'axe d'insertion compris entre 0,2 et 2,5 mm, de façon plus préférée entre 0,4 mm et 1,5 mm, optionnellement d'environ 1 mm. Le rayon et la hauteur sont de préférence compris entre 0,5 et 1,5 mm, optionnellement ces données sont d'environ 0,9 mm et définissent donc un congé de section transverse circulaire sur les faces vestibulaire, linguale, mésiale et distale. De préférence, les pourcentages valent environ 50%. De préférence, l'angle d'orientation pour la zone marginale est compris entre 0 et 5°, de préférence il est d'environ 2°. De préférence, l'angle d'orientation pour la zone médiane est compris entre 15 et 30°, de préférence il est d'environ 20°. Optionnellement, la hauteur minimale associée à chaque zone est d'environ 0,1 mm.

Plus préférentiellement, selon ces modes de réalisation préféré de l'invention, les contraintes techniques comprennent une épaisseur minimale d'un matériau de conception de l'élément prothétique, et les premières données de décalage des faces dépendent de cette épaisseur minimale. Il est ainsi avantageusement tenu compte de la réduction que doit subir au minimum chaque face pour pouvoir restaurer la au moins une dent en plaçant l'élément prothétique vu que celui-ci doit avoir une certaine épaisseur de paroi externe minimale. Il est à noter que cette réalisation préféré n'est aucunement limitative du cas où l'élément prothétique est une facette, par exemple, destinée à être posée sur une des faces car dans ce cas, il peut être prévu des données de décalage approximativement nulles pour les autres faces, ce qui est logique étant donné que ces données sont générées algorithmiquement à l'étape (iii.2) suivant la donnée du type de préparation dentaire (une facette donc).

De préférence, les premières données de décalage sont déterminées de façon indépendante des rayons et des hauteurs associés aux congés. Ceci est important compte tenu du fait que la première surface conique définie ci-dessous à l'étape (iv.2) part d'une courbe extrêmale bordant les congés elliptiques et qu'une dépendance directe des premières données de décalage créerait des points de rupture entre ces congés et cette première surface conique.

De façon avantageuse et préférée, ces modes de réalisation préférés de l'étape (iii) ont un impact et une application directe sur l'étape (iv). De préférence, celle-ci comprend une sous-étape de génération algorithmique d'une représentation tridimensionnelle d'une réduction volumétrique de la au moins une dent. Cette sous-étape comprend de préférence les sous-étapes suivantes (découlant donc avantageusement de ces modes de réalisations préférés de l'étape (iii)) :
(iv.1) générer une surface marginale consistant en les congés elliptiques des faces vestibulaire, linguale, mésiale et distale de ladite au moins une dent ;
(iv.2) générer une première surface conique autour dudit axe d'insertion à partir d'une courbe extrêmale bordant la surface marginale générée à la sous-étape (iv.1), cette première surface conique s'étendant parallèlement à ladite zone marginale et présentant une inclinaison vers l'axe d'insertion d'un angle correspondant à l'angle d'orientation de réduction volumétrique de ladite zone marginale ;
(iv.3) générer une deuxième surface conique autour dudit axe d'insertion à partir d'une courbe extrêmale bordant la première surface conique générée à la sous-étape (iv.2), cette deuxième surface conique s'étendant parallèlement à ladite zone médiane et présentant une inclinaison vers l'axe d'insertion d'un angle correspondant à l'angle d'orientation de réduction volumétrique de ladite zone médiane ;
(iv.4)
   - calculer une deuxième donnée de décalage pour chaque point des zones marginale et médiane sur la représentation tridimensionnelle isolée de la au moins une dent, cette deuxième donnée de décalage correspondant à un déplacement de ce point vers ou à l'opposé de l'axe d'insertion pour déplacer ce point sur une des première ou deuxième surfaces coniques ;
   - définir une surface primaire de réduction en déplaçant chaque point des zones marginale et médiane vers l'axe d'insertion selon :
      - la deuxième donnée de décalage de ce point si elle correspond à un déplacement vers l'axe d'insertion et si elle plus grande que la première donnée de décalage de ce point,
      - la première donnée de décalage de ce point sinon ;
(iv.5) générer une surface occlusale à partir d'une courbe extrêmale bordant la surface primaire de réduction générée à la sous-étape (iv.4) par un déplacement de chaque point de la face occlusale sur la représentation tridimensionnelle isolée de la au moins une dent, le long de l'axe d'insertion selon la première donnée de décalage ;
(iv.6) lisser et/ou régulariser une surface totale consistant en l'assemblage des surfaces marginales, primaire de réduction et occlusale, cette surface totale présentant un plan tangent à la surface totale en une intersection avec l'axe d'insertion perpendiculaire à l'axe d'insertion.

Ce mode de réalisation préféré de l'étape (iv) tire pleinement profit des modes de réalisation de l'étape (iii). Ainsi, il suffit au praticien de réaliser l'étape (iii.1) pour générer algorithmiquement une représentation tridimensionnelle de la réduction volumétrique de la au moins une dent, et ce grâce aux avantageuses étapes algorithmiques (iii.2) et (iv.1) à (iv.6). En outre, il doit être souligné que ce mode de réalisation préféré de l'étape (iv) permet une réduction volumétrique adapté au type de préparation dentaire qui va bien au-delà d'un simple retrait volumétrique automatique par face. En effet, la présent invention se veut apporter une solution technique au placement d'un élément prothétique définitif et non une de réduction volumétrique grossière pour le place d'un élément prothétique temporaire. C'est ainsi qu'il est proposé dans cette étape (iv) la définition de deux surfaces coniques facile à concevoir pour un dentiste pour la réduction volumétrique successive sur les zones marginale et médiane, et d'ensuite évaluer, à la sous-étape (iv.4), que ces surfaces coniques sont convenable pour le placement d'un élément prothétique conformément aux premières données de décalage du protocole dentaire. Cette opération est effectuée point par point de représentation tridimensionnelle isolée. Ainsi, la surface primaire de réduction obtenue à l'étape (iv.4) comprend à la fois des points des surfaces coniques et des points déterminés par des déplacements selon les premières données de décalage, de façon à tenir à la fois compte d'une pratique simple et efficace ultérieure pour le dentiste, mais aussi de tout le protocole dentaire déterminé à l'étape (iii.3). Cette étape représente un compromis entre une efficacité de taille dentaire pour obtenir la réduction volumétrique souhaitée et la nécessité de respecter tous les paramètres techniques déterminés à l'étape (iii), épaulant ainsi de façon remarquablement avantageuse le prothésiste dentaire dans son travail par des techniques algorithmiques développée dans le cadre de l'invention.

La sous-étape (iv.6) joue également un rôle important car elle permet de lisser et régulariser les différentes jonctions entre les surfaces coniques, les autres portions de la surface primaire, la surface marginale et la surface occlusale, proposant de préférence d'aplanir et/ou de tronquer une région de la surface occlusale au niveau de son intersection avec l'axe d'insertion (de sorte que ledit plan tangent est perpendiculaire à l'axe d'insertion), évitant ainsi toute irrégularité à ce niveau, et facilitant tant la conception de l'élément prothétique que la réduction volumétrique à venir de la au moins une dent.

De préférence, l'angle d'orientation pour la zone marginale est compris entre 0 et 5°. La première surface conique est donc de façon approximative cylindrique. De préférence, l'angle d'orientation pour la zone médiane est compris entre 15 et 30°. La deuxième surface conique est donc visuellement conique et son assemblage avec la première surface conique le long de la courbe extrêmale les bordant forme une surface globalement cylindro-conique. Cette dernière est attachée à la surface marginale formée des congés des faces vestibulaire, linguale, mésiale et distale. Cette forme cylindro-conique est très avantageuse car elle offre un excellent ancrage et une grande stabilité à l'élément prothétique à venir, empêchant en particulier son basculement. Grâce aux paramètres du protocole dentaire et à la succession des étapes (iv.1) à (iv.6), la réduction volumétrique obtenue à partir de la forme cylindro-conique est en outre paramétrée par face ce qui permet de privilégier l'esthétique, la stabilité ou la résistance de l'élément prothétique à venir selon la face considérée. Par exemple, l'esthétique pourra être privilégié pour une face vestibulaire, la stabilité pour une face linguale (ou palatine), etc. Ceci s'applique à d'autres sous-étapes. Par exemple, pour la face occlusale, l'esthétique et/ou la résistance pourront également être privilégiés, en prévoyant notamment suffisamment de place au matériau constituant l'élément prothétique. Une réduction volumétrique de la face occlusale sera de préférence conique.

La méthode de conception selon l'invention comprend en outre l'étape:
(vii') générer un quatrième fichier informatique sur base du deuxième fichier informatique validé et/ou modifié, le quatrième fichier informatique comprenant des instructions d'usinage de la au moins une dent correspondant à la représentation tridimensionnelle de la réduction volumétrique de la au moins une dent.

Ce quatrième fichier informatique est préférentiellement aussi un fichier CAM (d'usinage), généré sur base du deuxième fichier informatique de la même façon que ce que le troisième fichier informatique est généré à l'étape (vii), mais en tenant pleinement compte de protocole dentaire pour une taille de la au moins une dent.

Le quatrième fichier informatique a un grand avantage sur le deuxième fichier informatique de comprendre directement des instructions d'usinage de la au moins une dent utilisable par une machine d'usinage appropriée et/ou un praticien. Il est ainsi plus aisé de suivre les instructions d'usinage que de définir soi-même un usinage à partir d'un fichier CAD de représentation de réduction volumétrique de la au moins une dent. Ce quatrième fichier informatique constitue un élément informatique majeur de l'invention car il propose un usinage exacte de la au moins une dent permettant d'obtenir une surface externe usinée de la au moins une dent correspondant exactement à l'intrados de l'élément prothétique. Il est ainsi possible d'éviter tout espace vide entre la au moins une dent taillée et l'élément prothétique. La présence d'un tel espace vide serait néfaste à l'intégrité dentaire du patient car elle permettrait une prolifération de bactéries.

Selon un mode de réalisation préféré de la méthode de conception, elle comprend en outre les étapes supplémentaires suivantes :
(vii") générer un cinquième fichier informatique sur base dudit deuxième fichier informatique validé et/ou modifié, ledit cinquième fichier informatique comprenant des informations relatives à la représentation tridimensionnelle de l'intrados dudit élément prothétique obtenue à l'étape (vi), ces informations comprenant des instructions d'usinage d'un matériau brut rigide correspondant à ladite représentation tridimensionnelle de l'intrados dudit élément prothétique ;
(viii") usiner un bloc dudit matériau brut rigide sur base des instructions d'usinage dudit cinquième fichier informatique, de façon à produire une clé de contrôle d'un usinage de ladite au moins une dent correspondant à un usinage selon ladite représentation tridimensionnelle de la réduction volumétrique de ladite au moins une dent.

Ce cinquième fichier informatique est préférentiellement aussi un fichier CAM, optionnellement de stéréolithographie ou d'usinage, généré sur base du deuxième fichier informatique de la même façon que ce que le troisième fichier informatique est généré à l'étape (vii). Ce nouveau fichier permet de concevoir avantageusement une clé de contrôle pour le praticien lors de la préparation dentaire. En effet, il peut ainsi appliquer la clé de contrôle sur la au moins une dent de façon à vérifier si sa taille correspond bien à la réduction volumétrique attendue (celle-ci définissant l'intrados de l'élément prothétique). Bien que des techniques d'usinage soient mentionnées pour la production de la clé de contrôle, une production de celle-ci par des techniques d'impression tridimensionnelle (et une reformulation des étapes (vii") et (viii") en ce sens) ne se départirait aucunement du cadre de l'invention. Préférentiellement, selon le mode de réalisation comprenant une production d'une clé de contrôle, la méthode comprend en outre l'étape supplémentaire de couper la clé de contrôle en tranches parallèlement à un plan. Ainsi, le praticien peut enlever des tranches de la clé de façon à se rendre compte plus facilement de la correspondance entre sa taille et la réduction volumétrique attendue par vue sectionnelle. Ces tranches sont de préférences réalisées horizontalement (selon les surfaces occlusales des dents) ou verticalement (selon l'axe d'insertion).

Selon un mode de réalisation préféré de la méthode de conception comprenant les étapes (iv.1) à (iv.6), elle comprend en outre les étapes supplémentaires suivantes :
(iv') générer un sixième fichier informatique sur base dudit premier fichier informatique et dudit protocole dentaire validé et/ou modifié à l'étape (iii.3), le sixième fichier informatique comprenant trois collections d'instructions d'usinage d'un matériau brut rigide, chacune de ces collections comprenant des instructions d'usinage pour créer une cavité dans le matériau brut rigide correspondant à la représentation tridimensionnelle isolée,
   une première des collections d'instructions comprenant en outre des instructions d'usinage pour créer une fenêtre d'accès au moins partiellement conique autour la cavité suivant les première et deuxième surfaces coniques,
   une deuxième des collections d'instructions comprenant en outre des instructions d'usinage pour créer deux fenêtres d'accès supérieur à la cavité bordant des faces mésiale et distale de la cavité qui correspondent aux faces mésiale et distale de ladite au moins une dent sur la représentation tridimensionnelles isolée,
   une troisième des collections d'instructions comprenant en outre des instructions d'usinage d'une portion du matériau brut rigide entourant une zone médiane de la cavité correspondant à la zone médiane de ladite au moins une dent sur la représentation tridimensionnelle isolée, pour créer deux bords en pente selon l'angle d'orientation de réduction volumétrique de la zone médiane ;
(viii') usiner un premier, un deuxième et un troisième blocs dudit matériau brut rigide respectivement sur base des première, deuxième et troisième collections d'instructions d'usinage dudit sixième fichier informatique, de façon à produire trois guides d'usinage de ladite au moins une dent selon ladite représentation tridimensionnelle de la réduction volumétrique de ladite au moins une dent.

Ce sixième fichier informatique est préférentiellement aussi un fichier CAM, optionnellement de stéréolithographie ou d'usinage. Ce nouveau fichier permet de concevoir avantageusement des guides d'usinage pour le praticien lors de la préparation dentaire qui sont tout spécifiquement adaptés pour permettre au praticien de reproduire les sous-étapes de l'étape (iv) et ainsi d'obtenir une (représentation tridimensionnelle de la) réduction volumétrique de la au moins une dent fidèle à celle validée et/ou modifiée à l'étape (v). Les guides d'usinage obtenus à partir des premier, deuxième et troisième blocs sont de préférence à utiliser dans cet ordre de façon à reproduire plus ou moins directement certaines sous-étapes (ou produit de sous-étapes) de l'étape (iv). Bien que des techniques d'usinage soient mentionnées pour la production des guides, une production de ceux-ci par des techniques d'impression tridimensionnelle (et une reformulation des étapes (iv') et (viii') en ce sens) ne se départirait aucunement du cadre de l'invention. Le matériau brut rigide est de préférence au moins en partie et/ou au moins localement transparent. Optionnellement, il est totalement transparent. Optionnellement, il est au moins partiellement coloré. Préférentiellement, il est transparent localement autour de la cavité pour faciliter la visualisation de la au moins une dent à tailler. De préférence, les guides comprennent également des marquages comprenant des instructions de taille de la au moins une dent au moins autour de la cavité. De préférence, selon le mode de réalisation comprenant une production des guides, la méthode comprend en outre l'étape supplémentaire la solidarisation d'au moins une butée de sécurité et d'un rail de positionnement d'une fraise dentaire sur les premier, deuxième et troisième blocs. Ainsi, des erreurs de manipulation sont évitées durant la préparation dentaire grâce aux guides très avantageusement prévus dans le cadre de l'invention.

Dans le cadre du présent document, un « élément prothétique » fait préférentiellement référence à un élément parmi : une couronne, un pont, une obturation, un inlay, un onlay, un implant dentaire, une facette, ou une combinaison de plusieurs de ces éléments. Le terme d'élément prothétique englobe tout type de prothèses fixes, et tout type de prothèses amovibles partiellement ou complètement sur au moins une dent et/ou sur au moins un implant dentaire. Bien qu'une partie du présent document soit tout particulièrement illustré sur base d'une restauration dentaire comprenant une conception et/ou une pose d'une couronne, une restauration dentaire au moyen d'un autre élément prothétique ne saurait se départir du cadre de la méthode de conception de l'invention. Dans le cadre du présent document, les termes de « intrados » et « extrados » d'un élément prothétique font référence respectivement à une surface intérieure et une surface extérieure de l'élément prothétique, et sont connus d'un homme du métier prothésiste dentaire et/ou dentiste.

Dans le cadre de ce document, les termes de « dentiste », « chirurgien-dentiste », ou plus généralement de « praticien » sont de préférence utilisés de façon interchangeables. Le praticien responsable d'une capture d'une représentation tridimensionnelle intra-oral et/ou d'une image radiographique est préférentiellement un dentiste. Le praticien responsable d'une taille de la au moins une dent est préférentiellement un dentiste ou chirurgien-dentiste. Un praticien responsable de la réalisation des étapes (ii) à (vii) est préférentiellement un prothésiste dentaire, spécialisé dans la conception numérique d'éléments prothétiques.

Dans le cadre de ce document, le format STL est un format de fichier informatique connu d'un homme du métier. L'abréviation STL correspond à « Standard Triangle Language » en anglais.

Dans le cadre du présent document, un « congé » est une forme clinique de limite cervicale d'une préparation coronaire périphérique caractérisée par un profil légèrement concave formant un angle de raccordement obtus entre la zone coronaire préparée et la zone non préparée.

Dans le cadre du présent document, la au moins une dent est optionnellement une dent. De façon générale, la méthode s'applique de façon successive à chaque dent lorsqu'il y en a plusieurs. L'anatomie de la couronne d'une dent est divisée en cinq faces connues d'un homme du métier et dites :
- face « occlusale » qui est la face sur laquelle on mord ;
- face « vestibulaire » qui est la face se trouvant à l'extérieur, contre la joue ;
- face « linguale » (parfois appelée distinctivement face palatine pour les dents supérieures) qui est la face à l'intérieur orientée vers le palais et/ou que la langue effleure habituellement ;
- face « mésiale » qui est la face cachée entre deux dents la plus proche de l'axe médian de l'arcade dentaire ;
- face « distale » qui est la face cachée entre deux dents la plus éloignée de l'axe médian de l'arcade dentaire.

L'usage, dans le présent document, du verbe « comprendre » de ses variantes, ainsi que ses conjugaisons, ne peut en aucune façon exclure la présence d'éléments autres que ceux mentionnés. L'usage, dans le présent document, de l'article indéfini « un », « une », ou de l'article défini « le », « la » ou « l' », pour introduire un élément n'exclut pas la présence d'une pluralité de ces éléments.

Un élément prothétique produit par la méthode de conception selon l'invention est de préférence constitué d'un matériau de biocéramique pour assurer une compatibilité biologique avec les tissus mous et durs, et la dentition d'un patient, ainsi qu'une très grande longévité de l'élément prothétique.

Le présent document propose également une clé de contrôle produite par les modes de réalisation préféré de la méthode de conception comprenant l'étape (viii"). L'ensemble des modes de réalisation préférés ainsi que l'ensemble des avantages de ces modes de réalisation se transposent mutatis mutandis à la présente clé de contrôle. Il est également proposé trois guides d'usinage produits par les modes de réalisation préféré de la méthode de conception comprenant l'étape (viii'). L'ensemble des modes de réalisation préférés ainsi que l'ensemble des avantages de ces modes de réalisation se transposent mutatis mutandis aux présents guides. De préférence la clé de contrôle et/ou les guides sont constitués de résine rigide, préférentiellement transparente.

Il est aussi proposé des outils pour mettre en oeuvre la méthode de conception selon l'invention : un ensemble d'appareils, des programmes d'ordinateurs et des supports lisibles par un ordinateur. Ces outils sont détaillés ci-dessous.:
- un premier programme d'ordinateur comprenant des premières instructions qui, lorsque le premier programme d'ordinateur est exécuté, conduisent à mettre en oeuvre l'étape (iv) de la méthode de conception selon l'invention ;
- un deuxième programme d'ordinateur comprenant des deuxièmes instructions qui, lorsque le deuxième programme d'ordinateur est exécuté, conduisent à mettre en oeuvre l'étape (vii) de la méthode de conception selon l'invention ;
- un troisième programme d'ordinateur comprenant des troisièmes instructions qui, lorsque le troisième programme d'ordinateur est exécuté, conduisent à mettre en oeuvre l'étape (vii') de la méthode de conception selon l'invention ;
- un quatrième programme d'ordinateur comprenant des quatrièmes instructions qui, lorsque le quatrième programme d'ordinateur est exécuté, conduisent à mettre en oeuvre l'étape supplémentaire (vii") de la méthode de conception selon un mode de réalisation préféré de l'invention ;
- un cinquième programme d'ordinateur comprenant des cinquièmes instructions qui, lorsque le cinquième programme d'ordinateur est exécuté, conduisent à mettre en oeuvre l'étape supplémentaire (iv') de la méthode de conception selon un mode de réalisation préféré de l'invention ;
- un sixième programme d'ordinateur comprenant des sixièmes instructions qui, lorsque le sixième programme d'ordinateur est exécuté, conduisent à mettre en oeuvre l'étape supplémentaire (iii.2) de la méthode de conception selon un mode de réalisation préféré de l'invention.

Le document propose également un ensemble de programmes d'ordinateur comprenant le premier et/ou le deuxième et/ou le troisième et/ou le quatrième et/ou le cinquième et/ou le sixième programmes d'ordinateur.

Il est également proposé un support lisible par un ordinateur sur lequel est enregistré au moins un programme d'ordinateur présent dans un ensemble de programmes d'ordinateur.

Tel que détaillé précédemment, les premier, deuxième, troisième, quatrième, cinquième et sixième programmes d'ordinateur. Ils permettent d'automatiser partiellement la conception d'un élément prothétique, d'assister numériquement un prothésiste dans la définition de l'élément prothétique, et de planifier intelligemment sa production. En particulier, les modes de réalisation associés à ces programmes tel que susmentionné ainsi que les avantages de ceux-ci ayant trait aux étapes associées (iii), (iv), (iv'), (vii), (vii') et (vii") se transposent aux programmes et ensembles de programmes, ainsi qu'au support lisible par un ordinateur.

La présente invention propose un ensemble d'appareils pour concevoir un élément prothétique par exécution de la méthode de conception selon l'invention, l'ensemble d'appareils comprenant :
- au moins un appareil d'imagerie pour fournir le premier fichier informatique de l'étape (i) de la méthode de conception ;
- des moyens pour exécuter les étapes d'identification et de superposition des axes de référence communs de l'étape (i);
- un système informatique pour exécuter les étapes (ii)-(vii) et (vii') de la méthode de conception, comprenant :
   - une interface pour recevoir :
      au moins un paramètre technique déterminé à l'étape (iii) de la méthode de conception, et
      des validations et/ou des modifications du deuxième fichier informatique de l'étape (v) de la méthode de conception ;
      et pour visualiser et/ou communiquer des données sur :
         la représentation tridimensionnelle intra-orale et l'image radiographique du premier fichier informatique (11) fourni à l'étape (i)
         la représentation tridimensionnelle de l'extrados de l'élément prothétique obtenue à l'étape (ii) ;
         la représentation tridimensionnelle de la réduction volumétrique de la au moins une dent du deuxième fichier informatique généré à l'étape (iv) ;
         la représentation tridimensionnelle de la réduction volumétrique de la au moins une dent du deuxième fichier informatique validé et/ou modifié à l'étape (v) ;
         la représentation tridimensionnelle de l'intrados de l'élément prothétique obtenue à l'étape (vi) ;
   - une unité logique pour mettre en oeuvre les étapes (ii), (iv), (vi), (vii) et (vii') de la méthode de conception ;
- une machine de production pour lire les informations du troisième fichier informatique généré à l'étape (vii), et pour mettre en oeuvre l'étape (viii) de la méthode de conception.

Il est clair que divers avantages et modes de réalisation préférés de l'ensemble d'appareils selon l'invention se déduisent directement de l'ensemble des modes de réalisation préférés ainsi que l'ensemble des avantages de la méthode de conception selon l'invention. En particulier, le au moins un appareil d'imagerie comprend de préférence au moins un parmi : un scanner intra-oral et un appareil de radiographie par rayons X. Préférentiellement, les informations du troisième fichier informatique consiste en des instructions d'usinage d'un matériau et la machine de production comprend une machine d'usinage pour lire le troisième fichier informatique et exécuter ces instructions. Une telle machine d'usinage est connue d'un homme du métier et la fourniture d'instructions dans un fichier informatique compatible est l'objectif de l'étape (vii). Préférentiellement, la machine d'usinage est apte à concevoir plusieurs éléments prothétiques simultanément. Préférentiellement, le système informatique comprend un ordinateur dont un écran, un clavier et une souris sont l'interface et dont le processeur et au moins un support lisible sur lequel est enregistré au moins un programme d'ordinateur parmi les premier, deuxième et/ou troisième programmes d'ordinateur, définissent l'unité logique. Pour les modes de réalisation de l'invention approprié, l'unité logique est aussi de préférence également apte à mettre en oeuvre la sous-étape (iii.2) et les étapes (iv'), (vii') et (vii").

Le document propose un support lisible par ordinateur sur lequel est enregistré un deuxième fichier informatique généré à l'étape (iv) et/ou validé et/ou modifié à l'étape (v) de la méthode de conception selon l'invention. Le document propose également un support lisible par ordinateur sur lequel est enregistré au moins un parmi un troisième fichier informatique étant généré à l'étape (vii) de la méthode de conception selon l'invention, et/ou un quatrième fichier informatique étant généré à l'étape (vii') du mode de réalisation préféré de la méthode de conception selon l'invention.

Les avantages et modes de réalisation préférés de ces étapes (iv), (v), (vii) et (vii'), ainsi que des deuxième, troisième et quatrième fichiers informatiques se transposent mutatis mutandis aux présents supports lisibles par ordinateur. De préférence, le support lisible par ordinateur sur lequel est enregistré le troisième fichier informatique permet un transfert physique du troisième fichier informatique d'un centre informatique de données comprenant un système informatique tel que susmentionné pour l'exécution des étapes (ii) à (vii), et (vii') de la méthode de conception, vers un laboratoire comprenant une machine de production pour lire ce troisième fichier informatique et produire l'élément prothétique. De préférence, le support lisible par ordinateur sur lequel est enregistré le quatrième fichier informatique permet un transfert physique du quatrième fichier informatique du centre informatique de données, vers un cabinet dentaire ou un centre médical où un dentiste pourra le lire par ordinateur afin de s'informer des instructions d'usinage de la au moins une dent à exécuter.

Le document propose également un support lisible par ordinateur sur lequel est enregistré au moins un parmi un cinquième fichier informatique étant généré à l'étape (vii") de la méthode de conception selon l'invention, et/ou un sixième fichier informatique étant généré à l'étape (iv') du mode de réalisation préféré de la méthode de conception selon l'invention.

Les avantages et modes de réalisation préférés de ces étapes (iv') et (vii"), ainsi que des cinquième et sixième fichiers informatiques se transposent mutatis mutandis aux présents supports lisibles par ordinateur. De préférence, ce support lisible par ordinateur permet un transfert physique des cinquième et/ou sixième fichiers informatiques d'un centre informatique de données comprenant un système informatique tel que susmentionné pour l'exécution des étapes (ii) à (vii), (iv') et (vii") de la méthode de conception, vers une zone industrielle comprenant une machine de production pour lire ce cinquième et/ou sixième fichier informatique et produire la clé de contrôle et/ou les guides d'usinage.

L'invention comprend également un kit avec un système d'assistance d'un opérateur dans un placement d'un élément prothétique conçu par la méthode de conception, exploitant pleinement l'étape (vii') de la méthode selon l'invention. Ledit kit comprend l'ensemble d'appareils tel que défini précédemment (et correspondant à l'objet de la revendication 13) et un système d'assistance d'un opérateur dans une restauration dentaire qui est proposé dans le cadre de ce document comprend :
- un robot muni d'un bras robotisé mobile et solidaire d'un outil d'usinage placé en une extrémité du bras robotisé ;
- un système de guidage spatial du robot comprenant :
   - un premier repère spatial fixé au bras robotisé ;
   - un deuxième repère spatial configuré pour être attaché en un point d'une zone d'opération ;
   - un détecteur configuré pour déterminer une première distance séparant le détecteur du premier repère spatial, et une deuxième distance séparant le détecteur du deuxième repère spatial ;
   - une unité informatique logique configurée pour :
      lire un fichier informatique comprenant des instructions d'usinage d'au moins une dent,
      recevoir des données concernant les première et deuxième distances, et
      déterminer des informations de comparaison des données avec les instructions d'usinage ;
- un outil de communication connecté numériquement à l'unité informatique pour communiquer les informations de comparaison à l'opérateur.

Ce système d'assistance permet avantageusement à un opérateur, de préférence un dentiste et/ou chirurgien-dentiste, d'effectuer une taille de la moins une dent sans erreurs techniques.

En effet, l'outil de communication permet de communiquer à l'opérateur des informations de comparaison déterminées par l'unité informatique logique entre d'une part, des instructions d'usinage de la au moins une dent d'un fichier informatique, et d'autre part, une position relative entre le bras robotisé du robot et une zone d'opération, via des repères spatiaux dont il est évalué une distance les séparant par l'intermédiaire du détecteur. De cette façon, il peut être rendu compte d'écarts entre les instructions d'usinage prédéterminées et l'action réelle d'usinage via une position du bras robotisé. L'opérateur obtient ainsi un compte rendu de ses actions au moyen du robot de façon à suivre exactement les instructions d'usinage et à obtenir une taille précise et sans erreurs techniques.

Le système de guidage est une composante essentielle du système d'assistance car sans lui, il est impossible d'associer une action du robot et une instruction d'usinage en tenant pleinement compte de variation possible de position du patient et d'un repère attaché à la zone d'opération. Préférentiellement, le système de guidage permet d'informer en temps réel l'opérateur et/ou le robot d'un mouvement compensatoire à effectuer par rapport à un mouvement d'un patient, par exemple, dû à sa respiration, à des mouvements du maxillaire ou de la mandibule.

De préférence, de façon générale, par zone d'opération, il faut entendre une zone peu étendue d'une bouche d'un patient comprenant au moins une dent à restaurer.

Préférentiellement, le robot est connecté numériquement à l'unité informatique logique, de sorte que cette dernière est apte à contrôler une exécution des instructions d'usinage au moyen de l'outil d'usinage. Ainsi, et très avantageusement, le robot peut procéder automatiquement à la taille de la au moins une dent suivant les instructions d'usinage. Le robot est néanmoins préférentiellement semi-automatique dans le sens où il guide l'opérateur dans l'exécution de la taille de la au moins une dent. Ainsi, il assiste l'opérateur, par exemple, en le guidant par un mouvement et/ou une rotation et/ou une vibration du bras robotisé, ou encore en arrêtant automatiquement l'outil d'usinage en cas de sortie de la zone d'opération nuisible à l'intégrité physique d'un patient.

Avantageusement, le robot permet une précision inégalable seule par l'opérateur de l'ordre de 20 microns, ainsi qu'une réduction d'un temps d'intervention pour la taille de la au moins une dent de l'ordre de 40 à 60%. Avantageusement, le système d'assistance permet donc à l'opérateur d'effectuer une taille de la moins une dent avec une grande précision et sans fatigue mentale ou physique. Dans le cadre de ce document, la au moins une dent comprend optionnellement de façon générale au moins dix dents, et/ou au moins vingt-quatre dents d'un patient. Le système d'assistance permet avantageusement à l'opérateur d'effectuer la restauration d'un tel grand nombre de dents, en une séance, sans fatigue.

Préférentiellement, l'opérateur interagit avec le bras robotisé, en le sens que le bras robotisé est également apte à être guidé dans une certaine mesure par l'opérateur, par exemple pour être amené en zone d'opération. L'opérateur dispose également préférentiellement d'un action d'activation et de désactivation de l'outil d'usinage et/ou du robot. De façon combiné avec le mode de réalisation préféré précédent, le bras robotisé peut ainsi être amené dans la zone d'opération par l'opérateur, puis travailler de façon semi-autonome sous un contrôle d'exécution des instructions d'usinage par l'unité informatique logique. Avantageusement, cette double interaction du bras robotisé avec l'opérateur permet une grande flexibilité, une grande sécurité et une grande précision, pour l'opérateur dans une opération de restauration dentaire.

Préférentiellement, l'outil d'usinage est une fraise dentaire et le terme d'usinage fait référence à un fraisage.

Préférentiellement, le robot comprend six roulements pour subir des rotations partielles autour de six axes. Il comprend des roues pour se mouvoir sur un sol d'un cabinet dentaire.

Le fichier informatique en question consiste en un quatrième fichier informatique généré à l'étape (vii') selon la méthode de conception selon l'invention.

En particulier, dans ce cas, le système d'assistance permet à l'opérateur de tenir compte de la dentition globale et des tissus durs et mous d'un patient, la taille de la au moins une dent étant ainsi parfaitement adaptée aux besoins du patient et à un élément prothétique précédemment conçu par exécution de la méthode de conception selon l'invention. Le système d'assistance permet donc de mettre en oeuvre un processus de restauration dentaire précédemment défini dans ce résumé et duquel il est transposé les avantages au présent système d'assistance, le fichier informatique est un tel quatrième fichier informatique.

Selon un mode de réalisation préféré du système d'assistance, l'outil de communication comprend un écran pour afficher en temps réel, au moins partiellement, la zone d'opération et l'outil d'usinage. De préférence, les informations de comparaison comprennent en outre une position et/ou une orientation de l'outil d'usinage dans la zone d'opération.

Un tel outil de communication permet de guider l'opérateur dans une préparation dentaire en vue d'une restauration dentaire, par exemple, en guidant virtuellement une manipulation à effectuer sur l'écran ou en proposant plusieurs positions et/ou orientation de l'outil d'usinage dans la zone d'opération, ou même plusieurs trajectoires possibles de l'outil d'usinage dans la zone d'opération déterminée virtuellement via l'unité informatique logique sur base du fichier informatique.

De façon optionnelle, le deuxième repère spatial comprend au moins un parmi :
- une partie supérieure comprenant un casque et/ou au moins une sangle apte à être disposé autour d'un front d'un patient ;
- une partie inférieure comprenant une mentonnière apte à enfermer un menton d'un patient ;
et des deuxièmes symboles cibles détectables par le détecteur fixés sur la partie supérieure et/ou la partie inférieure.

Plus optionnellement, le deuxième repère spatial comprend les parties inférieure et supérieure. Avantageusement, le deuxième repère spatial permet de repérer individuellement une mâchoire supérieure et une mâchoire inférieure d'un patient. En effet, la partie supérieure suit les mouvements de la mâchoire supérieure couplée au front, alors que la partie inférieure suit les mouvements de la mâchoire inférieure couplée au menton. En outre, ce deuxième repère spatial peut de façon avantageuse optionnellement être utilisé pour une classe de patient.

Optionnellement, selon une nouvelle réalisation du deuxième repère spatial, celui-ci comprend un écarteur de joues d'un patient rigide apte à forcer une rigidification d'une bouche d'un patient, permettant un accès à la zone d'opération, comprenant des deuxièmes symboles cibles sur une portion supérieure et sur une portion inférieure, ces symboles étant apte à être détecté par le détecteur. Cette nouvelle réalisation optionnelle permet également un repérage des mâchoires inférieure et supérieure d'un patient via le détecteur.

Selon un mode de réalisation préféré du système d'assistance, le premier repère spatial comprend des premiers symboles cibles détectables par le détecteur ; et le deuxième repère spatial comprend :
- une partie dentaire apte à épouser au moins partiellement une forme d'une portion d'une dentition comprise dans la zone d'opération ;
- un élément protubérant comprenant des deuxièmes symboles cibles détectables par le détecteur, l'élément protubérant étant configuré pour être attaché à la partie dentaire au moyen d'un système d'attache, de façon à s'étendre hors de la zone d'opération.

Avantageusement, un tel deuxième repère spatial permet un usage d'une partie dentale propre à la dentition de chaque patient, alors que l'élément protubérant peut être réutilisé d'un patient à un autre et attacher au moyen du système d'attache sur une partie dentaire. Un autre avantage de la partie dentaire est qu'elle épouse au moins partiellement une forme de la portion de la dentition dans la zone d'opération, de sorte que l'ensemble du deuxième repère spatial est davantage stable par rapport au repère du patient, ce qui est primordiale dans l'objectif du système de guidage spatial du robot. Un deuxième repère spatial d'un autre type, par exemple, basé sur des symboles cibles qui seraient collés sur des dents de la dentition est également possible. Néanmoins, la caractéristique protubérante de l'élément protubérant est avantageuse car cet élément s'étend hors de la zone d'opération, ce qui facilite la détection des deuxièmes symboles cibles par le détecteur.

Selon une réalisation particulière, la partie dentaire est d'une forme hémicylindrique. La partie dentaire constitue alors une gouttière sensiblement courbe apte à être disposée sur la dentition.

Préférentiellement, la partie dentaire comprend une empreinte dentaire partielle conçu à partir d'une représentation tridimensionnelle intra-orale de la dentition, plus préférentiellement au moyen d'un scanner intra-oral. Plus préférentiellement, selon le mode de réalisation préféré pour lequel le fichier informatique est un quatrième fichier informatique obtenu selon une étape (vii') de la méthode de conception, la représentation tridimensionnelle intra-orale est celle fournie à l'étape (i). Compte tenu de l'usage temporaire de la partie dentaire, celle-ci peut-être réalisé en un matériau moins résistant que l'élément prothétique, et par exemple, au moyen de techniques d'usinage ou de processus de fabrication additive tel qu'une impression tridimensionnelle.

Indépendamment de cette mise en oeuvre semi-automatisée, comme précisé ci-dessus, le document propose également des dispositifs d'aide pour le un dentiste et/ou chirurgien-dentiste dans l'opération de taille de la moins une dent en diminuant le risque d'erreurs techniques. Dans ce contexte, le praticien est complètement maître de la pratique de la taille dentaire, sans aucune assistance par un robot, mais il peut néanmoins contrôler et/ou guider celle-ci par la clé de contrôle et/ou les guides

### Brève description des figures

D'autres caractéristiques et avantages de la présente invention apparaîtront à la lecture de la description détaillée qui suit pour la compréhension de laquelle on se reportera aux figures annexées parmi lesquelles :
- la figure 1 illustre une vue schématique un organigramme d'une méthode de conception d'un élément prothétique selon un mode de réalisation préféré de l'invention ;
- la figure 2 illustre une vue schématique d'une mise en oeuvre d'une méthode de restauration dentaire comprenant une méthode de conception d'un élément prothétique selon un mode de réalisation préféré de l'invention ;
- la figure 3 illustre une vue en perspective simplifiée d'une salle de dentisterie équipée avec un système d'assistance d'un opérateur dans une restauration dentaire décrit dans le résumé ;
- la figure 4 illustre une vue tridimensionnelle d'éléments d'un robot et d'un système de guidage spatial du robot décrit dans le résumé ;
- la figure 5 illustre une vue tridimensionnelle d'une clé de contrôle
- la figure 6 illustre une vue tridimensionnelle d'une utilisation de la clé de contrôle illustrée en figure 5 ;
- les figures 7 à 9 illustre une vue du dessus des guides
- la figure 10 illustre une vue tridimensionnelle d'un usage d'un guide;
- la figure 11 illustre une vue schématique d'une mise en oeuvre des étapes (iv.1) à (iv.6) d'une méthode de conception d'un élément prothétique selon un mode de réalisation préféré de l'invention ;
- la figure 12 illustre une application particulière des étapes (iv.1) à (iv.6) d'une méthode de conception selon un mode de réalisation préféré de l'invention.

Les dessins des figures ne sont pas à l'échelle. Généralement, des éléments semblables sont dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée

Les dessins ou figures décrits ne sont que schématiques et ne sont pas limitants.

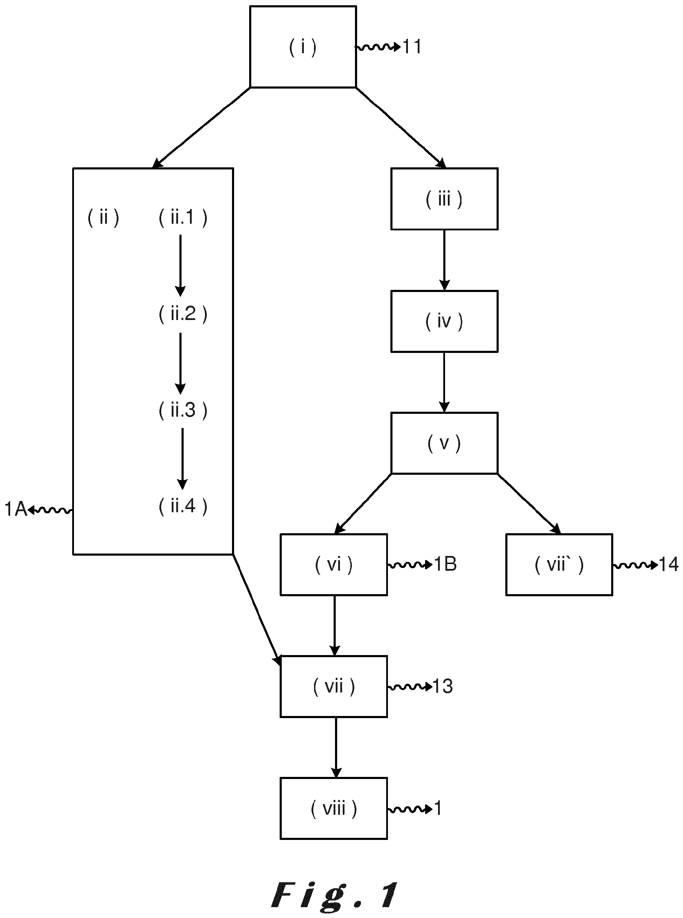

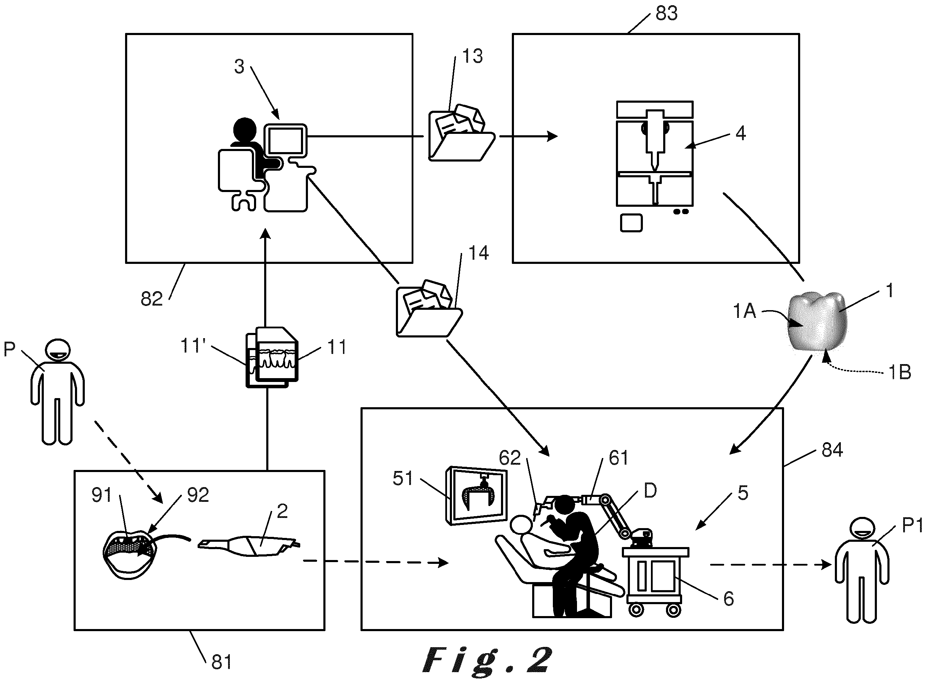

Les figures 1 et 2 illustrent au moins partiellement une mise en oeuvre d'une méthode de restauration dentaire d'une dent 91 d'une dentition 92 d'un patient P comprenant une méthode de conception d'un élément prothétique 1 selon un mode de réalisation préféré de l'invention. Il est ci-après fait référence aux notations (i), (ii), (iii), ... des différentes étapes de la méthode de conception introduites dans le résumé de l'invention ainsi que dans les revendications.

Il est supposé que le patient P doit subir une restauration dentaire comprenant un fraisage de la dent 91 et la pose d'un élément prothétique consistant en une couronne 1 sur la dent 91 fraisée. La méthode de conception selon l'invention est appliquée à la couronne 1 à concevoir. Cette méthode permet, selon un mode de réalisation préféré, d'être complété de façon à pouvoir restaurer facilement et rapidement la dent 91 du patient P.

Pour cela, la méthode propose au patient P de se rendre dans un centre d'imagerie tridimensionnelle 81 et de subir un scan intra-oral de sa dentition 92 au moyen d'un scanner intra-oral 2, et ce de façon à exécuter l'étape (i), c'est-à-dire, fournir un premier fichier informatique 11 comprenant une représentation tridimensionnelle intra-orale de la dentition 92 comprenant la dent 91 à restaurer au moyen de la couronne 1. Le centre d'imagerie tridimensionnelle 81 est, de façon préférée, un parmi : un cabinet dentaire, un centre médical, ou encore un centre d'imagerie de proximité. Avantageusement, un tel scan prend moins de cinq minutes pour être effectué ; après ce laps de temps, la patient P est libéré. Il est préférentiellement complété par une image radiographique panoramique bidimensionnelle de la bouche et/ou de la dentition 92 du patient P, et qui est soit jointe au premier fichier informatique 11, soit fournie simultanément au premier fichier informatique 11 dans un autre premier fichier informatique 11'. L'image radiographique panoramique est de préférence capturée au moyen d'un appareil à rayons X. Elle permet de fournir davantage d'information sur la structure de la dentition 92 du patient P, sur les nerfs, les tissus mous et durs associés, et ce notamment par une comparaison de la représentation tridimensionnelle intra-orale avec l'image radiographique.

Le premier fichier informatique 11 est ensuite envoyé, via un support lisible par ordinateur et/ou via un nuage de données partagées, dans un centre de données 82 comprenant un système informatique 3 permettant une exécution des étapes (ii) à (vii), et (vii') de la méthode de conception selon l'invention, dans l'ordre illustré en figure 1, et tel que détaillé dans le résumé de l'invention. Un prothésiste dentaire et/ou un designer CAD spécialisé traitent le premier fichier informatique 11 pour déterminer une représentation de la couronne 1, celle-ci comprenant des représentations tridimensionnelles 1A et 1B respectivement de son extrados et de son intrados, obtenues d'après les étapes (ii) et (vi) respectivement. Cette représentation est comprise dans un ou plusieurs fichiers informatiques de format STL de type CAD conçus au moins partiellement au moyen d'un premier programme d'ordinateur.

Un deuxième et un troisième programmes d'ordinateur permet d'utiliser des protocoles dentaires et de prothèse pour générer un troisième 13 et un quatrième 14 fichiers informatiques de type CAM qui comprennent des instructions d'usinage respectivement d'un matériau en céramique pour former la couronne 1, et de la dent 91 pour s'adapter à l'intrados 1B de la couronne 1 lors du placement.

Le troisième fichier informatique 13 est ensuite envoyé, via un support lisible par ordinateur et/ou via un nuage de données partagées, dans un laboratoire 83 comprenant une machine d'usinage 4 permettant une exécution de l'étape (viii) de la méthode de conception selon l'invention. En particulier, la machine d'usinage 4 lit le troisième fichier informatique 13 et exécute les instructions d'usinage de façon à usiner le matériau en céramique pour former la couronne 1, l'extrados et l'intrados de la couronne 1 consistant en des surfaces d'une forme correspondant aux représentations tridimensionnelles 1A et 1B.

Le quatrième fichier informatique 14 est, quant à lui, envoyé, via un support lisible par ordinateur et/ou un nuage de données partagées, dans un cabinet dentaire 84 d'un praticien chirurgien et/ou dentiste D équipé d'un système d'assistance 5 tel que décrit dans le résumé. Il est à noter qu'un praticien D possédant une telle machine d'usinage 4 dans le cabinet dentaire 84 peut recevoir le troisième fichier informatique 13 et produire lui-même la couronne 1 dont il a besoin. En particulier, les lieux 81, 83 et 84 sont susceptibles de coïncider.

Le praticien D, disposant du quatrième fichier informatique 14, réalise alors l'opération de restauration dentaire en fraisant la dent 91 et en fixant la couronne 1 sur la dent fraisée. Cette opération est bien connue, mais elle peut être sensiblement améliorée car la dent 91 est fraisée avec l'aide du système d'assistance 5 décrit dans le résumé et de façon à s'adapter à la couronne 1 déjà conçue, et non l'inverse. Ainsi, l'opération est d'une durée avantageuse de moins de trente minute et le patient P' retrouve un sourire à la sortie du cabinet dentaire.

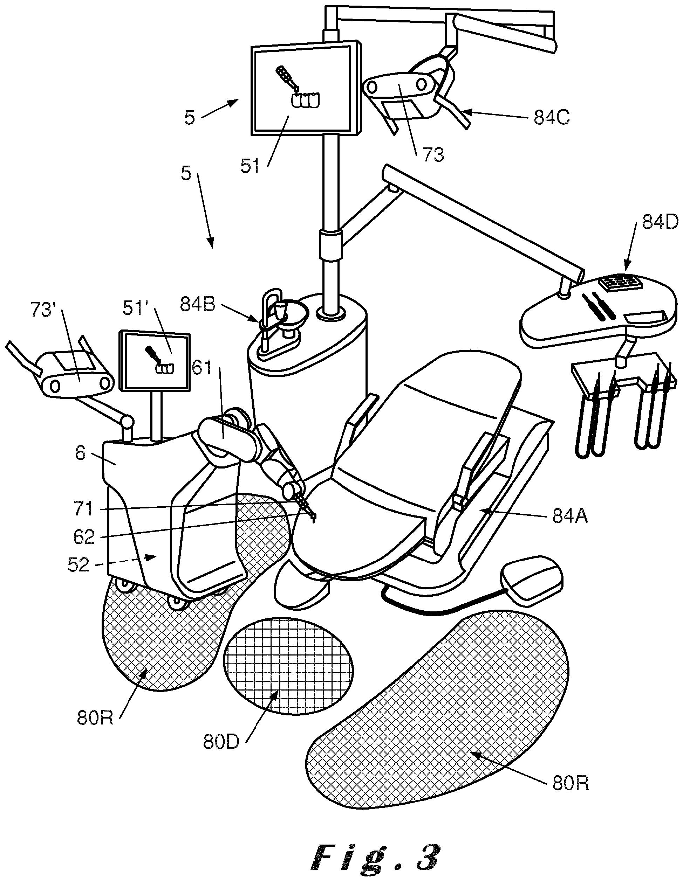

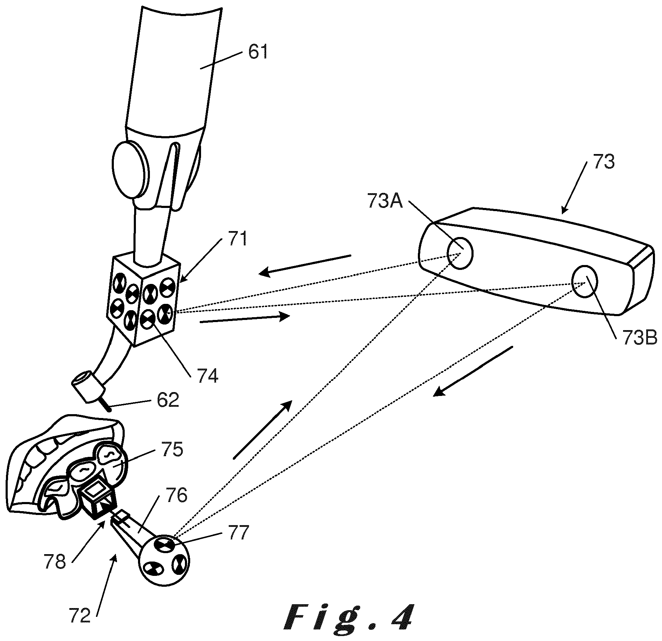

Le procédé menant à la restauration dentaire est maintenant décrit au regard de la figure 3 illustrant une vue simplifiée d'une salle d'opération du cabinet dentaire 84 équipée d'un système d'assistance 5 tel que décrit dans le résumé. Les éléments du cabinet dentaire 84 représentés en figure 2 sont tous illustrés en figure 3. La salle comprend des outils techniques classiques du praticien D tels qu'un siège 84A pour y accueillir le patient P, une station 84B comprenant une alimentation d'eau et une tour portant une lampe 84C placée en hauteur, et un bras mobile s'étendant autour du siège 84A, et auquel est couplé mécaniquement une palette 84D d'instruments standards du praticien D. Le système d'assistance 5 comprend un robot 6 mobile autour du siège 84A, dans une zone 80R. Il est muni de roues pour assurer cette mobilité. Il est également muni d'un bras articulé robotisé 61 mobile et solidaire d'un outil d'usinage consistant en une fraise 62 placée en une extrémité du bras robotisé 61. Le système d'assistance 5 comprend également un système de guidage spatial du robot 6 comprenant un détecteur 73, préférentiellement fixé à un support de la lampe 84C, en surplomb du siège 84A. Tel qu'illustré en figure 4, le détecteur 73 est configuré pour déterminer :
- une première distance séparant un de ses points d'au moins un parmi des premiers symboles cibles 74 d'un premier repère spatial 71 fixé au bras robotisé 61 ;
- une deuxième distance séparant le un de ses points d'au moins un parmi des deuxièmes symboles cibles 77 d'un deuxième repère spatial 72 qui est attaché en un point d'une zone d'opération de la dentition 92.
Pour permettre au détecteur 73 de déterminer ces première et deuxième distances avec profondeur, dans une direction quelconque de l'espace, il est prévu deux caméras 73A et 73B espacées sur le détecteur 73. Le deuxième repère spatial 72 comprend une partie dentaire 75 apte à épouser une forme d'une portion de la dentition 92 comprise dans la zone d'opération, cette portion ne comprenant pas la dent 91, et un élément protubérant 76 comprenant les deuxièmes symboles cibles 77. La partie dentaire 75 et l'élément protubérant sont pourvus d'un système d'attache 78 femme-mâle permettant d'emboîter par clips une extrémité mâle de l'élément protubérant 76 dans une extrémité femelle de la partie dentaire 75, et ce pour réutiliser avantageusement l'élément protubérant 76 d'une opération dentaire à l'autre, tout en jetant la partie dentaire 75 après une utilisation. De préférence, la partie dentaire est conçu d'un matériau solide et bon marché à partir du première fichier informatique 11 et ce pour constituer une empreinte dentaire partielle s'adaptant parfaitement à la portion de la dentition 92. La partie dentaire 75 est, par exemple, conçue par un processus de fabrication additive. Il est nécessaire de concevoir l'élément protubérant comme s'étendant hors de la zone d'opération pour facilité la visibilité des deuxièmes symboles cibles 77 par le détecteur 73 tout en garantissant que le deuxième repère spatial 72 rend compte des mouvement du patient P relativement à un repère fixe de la salle et au bras robotisé 61. Le système de guidage spatial comprend enfin une unité informatique logique 52 comprenant un processeur agencé au sein d'une carcasse du robot 6, de façon à limiter l'encombrement du système d'assistance 5. L'unité informatique logique 52 est configurée pour :
- lire le quatrième fichier informatique 14,
- recevoir des données concernant les première et deuxième distances du détecteur 73, et
- déterminer des informations de comparaison des données avec les instructions d'usinage du quatrième fichier informatique 14,
- contrôler et/ou arrêter le robot 6 et/ou la fraise 62 en fonction des informations de comparaison.
Le système d'assistance 5 comprend également un outil de communication 51 connecté numériquement à l'unité informatique 52 et comprenant un écran pour communiquer les informations de comparaison au praticien D. Ainsi, et tel que détaillé dans le résumé de l'invention, le praticien D peut être guidé dans son fraisage par le système d'assistance 5 de façon à garantir une grande fiabilité et une grande rapidité de l'opération, ainsi qu'une grande précision du fraisage, et en particulier, une obtention d'une surface de la dent 91 fraisée correspondant exactement à l'intrados de la couronne 1 à placer. Optionnellement, le robot 6 comprend un autre bras mécanique mobile comprenant un autre détecteur 73' d'une structure et pour un usage semblables au détecteur 73. Optionnellement, le robot 6 comprend un autre outil 51' d'une structure et d'un usage semblables à l'outil de communication 51. En particulier, il est connecté numériquement à l'unité informatique 52 pour communiquer et/ou recevoir les informations de comparaison au praticien D. Il consiste de préférence en un parmi un ordinateur portable, ou une tablette interactive, pour le praticien D. Durant l'opération de chirurgie dentaire, le praticien D agissant en tant opérateur assisté est essentiellement situé dans une zone 80D, entourée de façon latérale par la zone 80R.

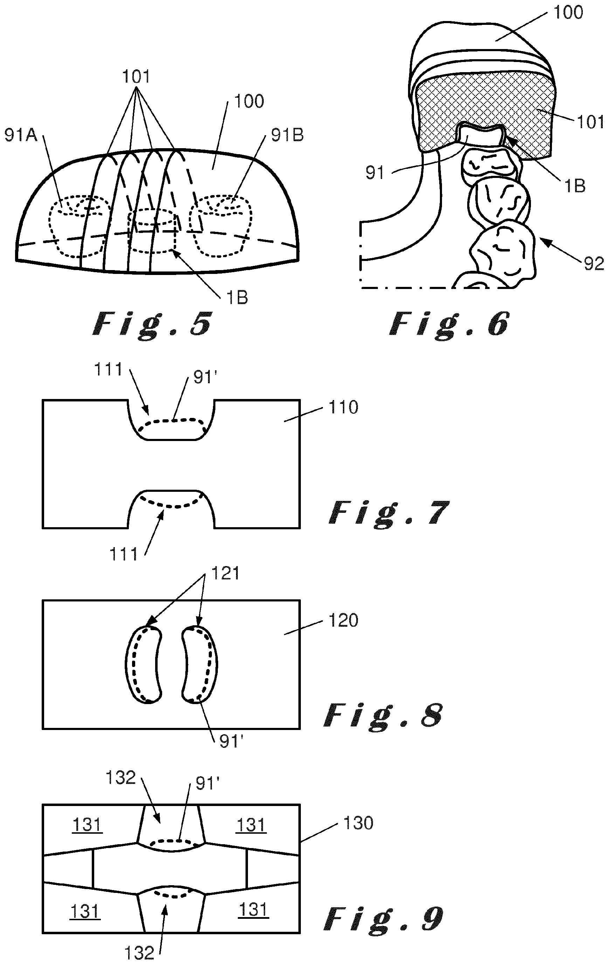

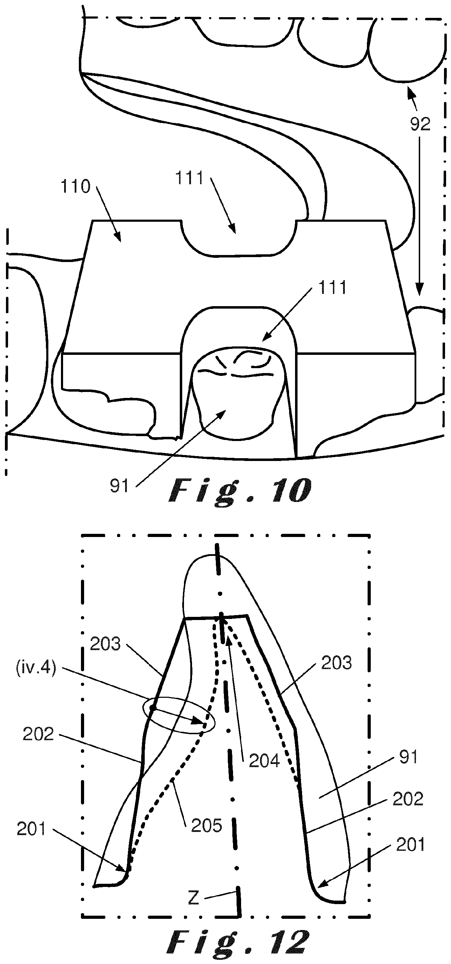

Le procédé menant à la restauration dentaire décrit au regard au regard des figures 3 et 4 est avantageusement (semi-)automatisé à l'aide du système d'assistance 5 tel que décrit dans le résumé. Toutefois, ce système d'assistance 5 est susceptible d'être coûteux et complexe à mettre en oeuvre. Il est donc proposé des alternatives peu coûteuses et très avantageuses de clé de contrôle et de guides pour l'usinage de la au moins une dent 91. Cette clé de contrôle 100 et ces guides 110, 120 et 130 sont introduits précisément dans le résumé de l'invention et illustrés aux figures 5 à 10. La clé de contrôle 100 est formée à partir d'un bloc de matériau brut rigide qui est usiné de sorte que la représentation tridimensionnelle de l'intrados 1B de l'élément prothétique 1 est formée à partir d'une face inférieure du bloc. De préférence, des représentations tridimensionnelles des dents voisines 91A et 91B sont également usinées de façon semblable. La clé de contrôle 100 fait donc office de moule qui pourra être placée sur la dentition 92 du patient lorsque la au moins une dent 91 aura été préparée par un praticien. La clé de contrôle 100 permet ainsi au praticien de se rendre compte d'éventuelles erreurs de taille de la au moins une dent 91. Pour faciliter cela, la clé de contrôle 100 est coupée en tranches 101 parallèlement à un plan (verticale dans le cas illustré, mais qui peut tout aussi bien être horizontal). De cette façon, le praticien peut déplacer certaines tranches 101 de façon à observer l'évolution de sa taille et sa bonne correspondance avec l'intrados 1B de l'élément prothétique 1. Ceci est en particulier illustré en figure 6. Les guides 110, 120 et 130 sont quant à eux directement associés à la pratique de l'usinage de la au moins une dent 91 au regard de la méthode comprenant des étapes (iv.1) à (iv.6) tel qu'il est expliqué dans le résumé de l'invention. Ils sont représentés vus du dessus (en aval de l'axe d'insertion lorsqu'ils sont disposés sur la au moins une dent 91) en figures 7 à 9 et sont obtenus respectivement par l'application des première, deuxième et troisième collections d'instructions d'usinage aux premier, deuxième et troisième blocs d'un matériau brut rigide transparent, tel qu'expliqué dans le résumé de l'invention. Chacun comprend une cavité 91' dans ce matériau brut correspondant à la représentation tridimensionnelle isolée de la au moins une dent 91 qui est obtenue par l'étape (iii.1). Des fenêtres 111, 121 et 132 sont pratiquées respectivement dans les guides 110, 120 et 130 pour avoir accès à cette cavité 91' selon des directions et angles d'attaque pour la taille dentaire adaptés pour reproduire au mieux certaines sous-étapes ou résultats résultants de sous-étapes de l'étape (iv). Le guide 130 est usiné de sorte que deux bords en pente 131 sont créés donnant une forme de toit à cette portion du bloc de matériau brut, les pentes étant déterminée selon un angle d'orientation de réduction volumétrique de la zone médiane de la au moins une dent 91 compris dans le protocole dentaire selon l'étape (iii). Le placement d'un tel guide 110 lors de la préparation dentaire est illustré en figure 10. Ces guides 110, 120, 130 guide l'usinage de la au moins une dent 91 pour reproduire en particulier la surface primaire selon la sous-étape (iv.4). D'autres tels guides peuvent être déduits des sous-étapes de l'étape (iv).

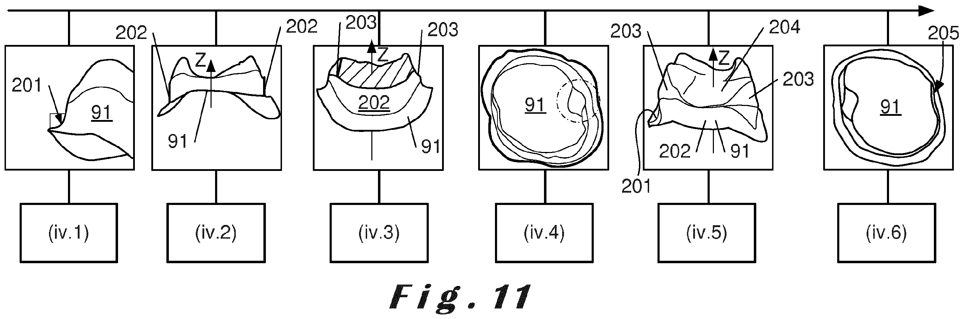

La décomposition de l'étape (iv) en les sous-étapes (iv.1) à (iv.6) est commenté dans le résumé de l'invention et illustré en figure 11 au regard d'une couronne, ce choix étant non limitatif car ces sous-étapes s'appliquent efficacement au cas d'autres éléments prothétiques 1, vu que ceux-ci influençant un choix d'axe d'insertion Z par le protocole dentaire selon l'étape (iii.1) à (iii.3). Ainsi, cette figure permet d'illustrer ces sous-étapes selon les références suivantes. Les congés elliptiques 201 sont générés à la sous-étape (iv.1) au niveau marginal de la au moins une dent 91, la première surface conique 202 (celle-ci étant approximativement cylindrique) est générée à l'étape (iv.2) autour de la zone marginale de la au moins une dent 91, la deuxième surface conique 203 est générée à l'étape (iv.3) autour de la zone médiane de la au moins une dent 91, l'étape (iv) permet de comparer les surfaces coniques 202 et 203 avec une surface de décalage qui serait obtenue en déplaçant chaque point des zones marginale et médiane vers l'axe d'insertion Z selon les premières données de décalage issues du protocole dentaire. En effet, il est possible pour des dents particulières que des points des surfaces coniques 202 et 203 soient en dehors de la (représentation tridimensionnelle de la) au moins une dent 91, de sorte que cela n'aurait plus aucun sens de définir une réduction volumétrique de la au moins une dent 91 sur cette base. Ceci est illustré dans la figure 12 en particulier. Il est donc nécessaire de projeter ces points à l'intérieur de la (représentation tridimensionnelle de la) au moins une dent 91. Cette projection doit se faire en tenant compte qu'il faudra en quelque sorte « rentrer suffisamment loin » dans la (représentation tridimensionnelle de la) au moins une dent 91 pour garantir assez de place pour l'élément prothétique 1, vu que celui-ci a une certaine épaisseur. C'est tout l'intérêt des premières données de décalage qui découle du protocole dentaire et encodent en quelque sorte de préférence cette épaisseur minimale qu'il convient d'enlever à chaque face de la au moins une dent 91 pour pouvoir placer l'élément prothétique 1 en tenant compte des tolérances associées à celui-ci. Ainsi l'étape (iv.4) propose cette avantageuse et judicieuse correction des surfaces coniques 202 et 203 pour permettre de définir une surface primaire de réduction convenable pour effectuer la réduction volumétrique de la au moins une dent 91. Ensuite, une surface occlusale réduite 204 est générée à l'étape (iv.5) et finalement une surface totale 205 finale est générée à l'étape (iv.6), celle-ci consistant en l'assemblage des congés 201, des surfaces primaire de réduction et occlusale 204 lissés et régularisés.

En résumé, l'invention concerne, une méthode de conception d'un élément prothétique 1 exécutable préalablement à une taille d'une dent 91 d'un patient pour la pose de l'élément prothétique 1.

La présente invention a été décrite en relation avec des modes de réalisations spécifiques, qui ont une valeur purement illustrative et ne doivent pas être considérés comme limitatifs. D'une manière générale, il apparaîtra évident pour un homme du métier que la présente invention n'est pas limités aux exemples illustrés et/ou décrits ci-dessus. L'étendue de la protection de l'invention est définie par les revendications.

## Revendications

1. Méthode de conception d'un élément prothétique (1) comprenant les étapes suivantes :
(i)
• fournir un premier fichier informatique (11) comprenant :
- une représentation tridimensionnelle intra-orale d'une dentition (92) comprenant au moins une dent (91) à restaurer au moyen dudit élément prothétique (1) ;
- une image radiographique de ladite dentition (92) ;
• identifier des axes de référence communs sur ladite représentation tridimensionnelle intra-orale et sur ladite image radiographique ;
• comparer ladite représentation tridimensionnelle intra-orale avec ladite image radiographique, cette sous-étape de comparaison comprenant une superposition desdits axes de référence communs ;
(ii) déterminer une représentation tridimensionnelle d'un extrados (1A) dudit élément prothétique (1) sur base dudit premier fichier informatique (11) ;
(iii) déterminer des paramètres techniques comprenant :
- un protocole dentaire, et/ou
- un type de préparation dentaire, et/ou
- des contraintes techniques,
sur base dudit premier fichier informatique (11), au moins un desdits paramètres techniques étant déterminé sur base de la sous-étape de comparaison de l'étape (i) ;
(iv) générer un deuxième fichier informatique comprenant une représentation tridimensionnelle d'une réduction volumétrique de ladite au moins une dent (91) sur base desdits paramètres techniques ;
(v) valider et/ou modifier ledit deuxième fichier informatique ;
(vi) obtenir une représentation tridimensionnelle d'un intrados (1B) dudit élément prothétique (1) sur base dudit deuxième fichier informatique validé et/ou modifié ;
(vii) générer un troisième fichier informatique (13) comprenant des informations relatives aux représentations tridimensionnelles desdits extrados (1A) et intrados (1B) dudit élément prothétique (1) ;
(vii') générer un quatrième fichier informatique (14) sur base dudit deuxième fichier informatique validé et/ou modifié, le quatrième fichier informatique (14) comprenant des instructions d'usinage de la au moins une dent (91) correspondant à la représentation tridimensionnelle de la réduction volumétrique de la au moins une dent (91) ;
(viii) produire ledit élément prothétique (1) sur base dudit troisième fichier informatique (13).

2. Méthode selon la revendication précédente, **caractérisée en ce que** l'étape (ii) comprend les sous-étapes suivantes :
(ii.1) choisir une représentation tridimensionnelle modèle d'une dentition modèle dans une base de données sur base dudit premier fichier informatique (11);
(ii.2) choisir une zone de la représentation tridimensionnelle modèle correspondant à une zone de la représentation tridimensionnelle intra-orale correspondant à ladite au moins une dent (91) ;
(ii.3) valider et/ou modifier la zone de la représentation tridimensionnelle modèle sur base dudit premier fichier informatique (11) ;
(ii.4) définir la représentation tridimensionnelle de l'extrados (1A) dudit élément prothétique (1) à partir de la zone validée et/ou modifiée de la représentation tridimensionnelle modèle.

3. Méthode selon l'une quelconque des revendications précédentes **caractérisée en ce que** l'étape (iii) comprend les sous-étapes suivantes :
(iii.1) déterminer un type de préparation dentaire sur base dudit premier fichier informatique (11) ;
(iii.2) générer algorithmiquement un protocole dentaire sur base du type de préparation dentaire déterminé à l'étape (iii.1), ledit protocole dentaire consistant en une collection de données numériques pour paramétrer géométriquement ladite représentation tridimensionnelle de la réduction volumétrique ;
(iii.3) valider et/ou modifier ledit protocole dentaire sur base de la sous-étape de comparaison de l'étape (i).

4. Méthode selon la revendication précédente,
**caractérisée en ce que** la sous-étape (iii.1) comprend les sous-étapes suivantes :
● visualiser ladite représentation tridimensionnelle intra-orale d'une dentition (92) ;
● segmenter ladite représentation tridimensionnelle intra-orale d'une dentition (92) de façon à obtenir une représentation tridimensionnelle isolée de la au moins une dent (91) ;
● générer algorithmiquement des faces vestibulaire, linguale, mésiale, distale et occlusale de la au moins une dent (91) par identification d'un point de chacune de ces faces au niveau de la représentation tridimensionnelle isolée de la au moins une dent (91) ;
● modifier et/ou valider des frontières desdites faces vestibulaire, linguale, mésiale, distale et occlusale de la au moins une dent (91) par des ajouts, des déplacements et/ou des retraits de points de ces faces sur la représentation tridimensionnelle isolée de la au moins une dent (91) ;
● identifier un repère d'au moins un desdits axes de référence sur base de la sous-étape de comparaison de l'étape (i), le repère comprenant un axe d'insertion de la au moins une dent (91).

5. Méthode selon la revendication précédente, **caractérisée en ce que** les données numériques de la sous-étape (iii.2) comprennent :
- pour chaque face parmi les faces vestibulaire, linguale, mésiale et distale de ladite au moins une dent (91) :
● un rayon ;
● une hauteur ;
correspondant à une paramétrisation d'une section transverse d'un congé elliptique de la face selon un arc d'ellipse de demi grand-axe correspondant audit rayon mesuré essentiellement perpendiculairement audit axe d'insertion et de demi petit-axe correspondant à ladite hauteur mesurée de façon essentiellement parallèle audit axe d'insertion ;
- pour chaque face parmi les faces vestibulaire, linguale, mésiale et distale de ladite au moins une dent (91) :
une première donnée de décalage correspondant à un déplacement de chaque point de la face sur la représentation tridimensionnelle isolée de la au moins une dent vers l'axe d'insertion ;
- pour la face occlusale de ladite au moins une dent (91) :
une première donnée de décalage correspondant à un déplacement de chaque point de la face occlusale sur la représentation tridimensionnelle isolée de la au moins une dent le long de l'axe d'insertion ;
- deux pourcentages définissant deux zones consistant en des zones marginale et médiane de ladite au moins une dent (91) sur la représentation tridimensionnelles isolée de la au moins une dent (91), chaque pourcentage correspondant au rapport entre une hauteur d'une zone et une hauteur des deux zones, ces hauteurs étant mesurées essentiellement parallèlement à l'axe d'insertion ;
- pour chacune desdites zones :
● un angle d'orientation de réduction volumétrique mesuré par rapport à l'axe d'insertion ;
● une hauteur minimale.

6. Méthode selon la revendication précédente, **caractérisée en ce que** les contraintes techniques comprennent une épaisseur minimale d'un matériau de conception de l'élément prothétique (1), et **en ce que** les premières données de décalage des faces dépendent de cette épaisseur minimale.

7. Méthode selon l'une quelconque des deux revendications précédentes, **caractérisée en ce que** l'étape (iv) comprend une sous-étape de génération algorithmique d'une représentation tridimensionnelle d'une réduction volumétrique de ladite au moins une dent (91), cette sous-étape comprenant les sous-étapes suivantes :
(iv.1) générer une surface marginale consistant en les congés elliptiques (201) des faces vestibulaire, linguale, mésiale et distale de ladite au moins une dent (91) ;
(iv.2) générer une première surface conique (202) autour dudit axe d'insertion (Z) à partir d'une courbe extrêmale bordant la surface marginale générée à la sous-étape (iv.1), cette première surface conique s'étendant parallèlement à ladite zone marginale et présentant une inclinaison vers l'axe d'insertion d'un angle correspondant à l'angle d'orientation de réduction volumétrique de ladite zone marginale ;
(iv.3) générer une deuxième surface conique (203) autour dudit axe d'insertion à partir d'une courbe extrêmale bordant la première surface conique générée à la sous-étape (iv.2), cette deuxième surface conique s'étendant parallèlement à ladite zone médiane et présentant une inclinaison vers l'axe d'insertion d'un angle correspondant à l'angle d'orientation de réduction volumétrique de ladite zone médiane ;
(iv.4)
• calculer une deuxième donnée de décalage pour chaque point des zones marginale et médiane sur la représentation tridimensionnelle isolée de la au moins une dent, cette deuxième donnée de décalage correspondant à un déplacement de ce point vers ou à l'opposé de l'axe d'insertion pour déplacer ce point sur une des première ou deuxième surfaces coniques ;
• définir une surface primaire de réduction en déplaçant chaque point des zones marginale et médiane vers l'axe d'insertion selon :
- la deuxième donnée de décalage de ce point si elle correspond à un déplacement vers l'axe d'insertion et si elle plus grande que la première donnée de décalage de ce point,
- la première donnée de décalage de ce point sinon ;
(iv.5) générer une surface occlusale (204) à partir d'une courbe extrêmale bordant la surface primaire de réduction générée à la sous-étape (iv.4) par un déplacement de chaque point de la face occlusale sur la représentation tridimensionnelle isolée de la au moins une dent, le long de l'axe d'insertion selon la première donnée de décalage ;
(iv.6) lisser et/ou régulariser une surface totale (205) consistant en l'assemblage des surfaces marginales, primaire de réduction et occlusale, cette surface totale présentant un plan tangent à la surface totale en une intersection avec l'axe d'insertion perpendiculaire à l'axe d'insertion.

8. Méthode selon l'une quelconque des revendications précédentes, **caractérisée en ce que** :
- ledit deuxième fichier informatique consiste en un fichier de format STL modifiable ;
- l'étape (v) comprend une validation et/ou une modification de chacun parmi des paramètres géométriques relatifs à ladite représentation tridimensionnelle de la réduction volumétrique de ladite au moins une dent (91) dans un ensemble de valeurs admissibles préalablement défini par au moins un desdits paramètres techniques déterminé à l'étape (iii).

9. Méthode de conception selon l'une quelconque des revendications précédentes **caractérisée en ce que** :
- lesdites informations dudit troisième fichier informatique (13) comprennent des instructions d'usinage d'un matériau ;
- l'étape (viii) comprend une sous-étape d'usinage dudit matériau sur base desdites instructions d'usinage.

10. Méthode de conception selon l'une quelconque des revendications précédentes, comprenant en outre les étapes supplémentaires suivantes :
(vii") générer un cinquième fichier informatique sur base dudit deuxième fichier informatique validé et/ou modifié, ledit cinquième fichier informatique comprenant des informations relatives à la représentation tridimensionnelle de l'intrados (1B) dudit élément prothétique (1) obtenue à l'étape (vi), ces informations comprenant des instructions d'usinage d'un matériau brut rigide correspondant à ladite représentation tridimensionnelle de l'intrados (1B) dudit élément prothétique (1) ;
(viii") usiner un bloc dudit matériau brut rigide sur base des instructions d'usinage dudit cinquième fichier informatique (13), de façon à produire une clé de contrôle (100) d'un usinage de ladite au moins une dent (91) correspondant à un usinage selon ladite représentation tridimensionnelle de la réduction volumétrique de ladite au moins une dent (91) ;
et comprenant en outre l'étape supplémentaire de couper la clé de contrôle (100) en tranches (101) parallèlement à un plan.

11. Méthode de conception selon la revendication 7, comprenant en outre les étapes supplémentaires suivantes :
(iv') générer un sixième fichier informatique sur base dudit premier fichier informatique (11) et dudit protocole dentaire validé et/ou modifié à l'étape (iii.3), le sixième fichier informatique comprenant trois collections d'instructions d'usinage d'un matériau brut rigide, chacune de ces collections comprenant des instructions d'usinage pour créer une cavité (91') dans le matériau brut rigide correspondant à la représentation tridimensionnelle isolée,
une première des collections d'instructions comprenant en outre des instructions d'usinage pour créer une fenêtre (111) d'accès au moins partiellement conique autour la cavité (91') suivant les première et deuxième surfaces coniques,
une deuxième des collections d'instructions comprenant en outre des instructions d'usinage pour créer deux fenêtres (121) d'accès supérieur à la cavité (91') bordant des faces mésiale et distale de la cavité qui correspondent aux faces mésiale et distale de ladite au moins une dent (91) sur la représentation tridimensionnelles isolée,
une troisième des collections d'instructions comprenant en outre des instructions d'usinage d'une portion du matériau brut rigide entourant une zone médiane de la cavité correspondant à la zone médiane de ladite au moins une dent (91) sur la représentation tridimensionnelle isolée, pour créer deux bords en pente (131) selon l'angle d'orientation de réduction volumétrique de la zone médiane ;
(viii') usiner un premier, un deuxième et un troisième blocs dudit matériau brut rigide respectivement sur base des première, deuxième et troisième collections d'instructions d'usinage dudit sixième fichier informatique, de façon à produire trois guides (110, 120, 130) d'usinage de la au moins une dent (91) selon ladite représentation tridimensionnelle de la réduction volumétrique de la au moins une dent (91).

12. Méthode de conception selon la revendication précédente, **caractérisé en ce qu'**elle comprend en outre l'étape supplémentaire la solidarisation d'au moins une butée de sécurité et d'un rail de positionnement d'une fraise dentaire sur les guides (110, 120, 130).

13. Ensemble d'appareils pour concevoir un élément prothétique (1) par exécution de la méthode de conception selon l'une quelconque des revendications précédentes, l'ensemble d'appareils comprenant :
- au moins un appareil d'imagerie (2) pour fournir le premier fichier informatique de l'étape (i) de la méthode de conception ;
- des moyens pour exécuter les étapes d'identification et de superposition des axes de référence communs de l'étape (i) et
- un système informatique (3) pour exécuter les étapes (ii) à (vii), et (vii') de la méthode de conception, comprenant :
• une interface pour recevoir :
au moins un paramètre technique déterminé à l'étape (iii) de la méthode de conception, et
des validations et/ou des modifications du deuxième fichier informatique de l'étape (v) de la méthode de conception ;
et pour visualiser et/ou communiquer des données sur :
la représentation tridimensionnelle intra-orale et l'image radiographique du premier fichier informatique (11) fourni à l'étape (i) ;
la représentation tridimensionnelle de l'extrados (1A) dudit élément prothétique (1) obtenue à l'étape (ii) ;
la représentation tridimensionnelle de la réduction volumétrique de la au moins une dent (91) du deuxième fichier informatique généré à l'étape (iv) ;
la représentation tridimensionnelle de la réduction volumétrique de la au moins une dent (91) du deuxième fichier informatique validé et/ou modifié à l'étape (v) ;
la représentation tridimensionnelle de l'intrados (1B) dudit élément prothétique (1) obtenue à l'étape (vi) ;
• une unité logique pour mettre en oeuvre les étapes (ii), (iv), (vi), (vii) et (vii') de la méthode de conception ;
- une machine de production (4) pour lire les informations du troisième fichier informatique (13) généré à l'étape (vii), et pour mettre en oeuvre l'étape (viii) de la méthode de conception.

14. Ensemble d'appareils selon la revendication précédente,
dans lequel le système informatique (3) comprend un ordinateur et dans lequel l'unité logique est définie par :
- un processeur de l'ordinateur et
- au moins un support lisible par l'ordinateur sur lequel sont enregistrés :
• un premier programme d'ordinateur comprenant des premières instructions qui, lorsque ledit premier programme d'ordinateur est exécuté, conduisent à mettre en oeuvre l'étape (iv) de la méthode de conception selon l'une quelconque des revendications 1 à 12 ;
• un deuxième programme d'ordinateur comprenant des deuxièmes instructions qui, lorsque ledit deuxième programme d'ordinateur est exécuté, conduisent à mettre en oeuvre l'étape (vii) de la méthode de conception selon l'une quelconque des revendications 1 à 12 ; et
• un troisième programme d'ordinateur comprenant des troisièmes instructions qui, lorsque ledit troisième programme d'ordinateur est exécuté, conduisent à mettre en oeuvre l'étape (vii') de la méthode de conception selon l'une quelconque des revendications 1 à 12.

15. Kit comprenant un ensemble d'appareils selon la revendication 13 ou 14, et un système d'assistance (5) d'un opérateur (D) dans une restauration dentaire comprenant :
- un robot (6) muni d'un bras robotisé (61) mobile et solidaire d'un outil d'usinage (62) placé en une extrémité dudit bras robotisé (61) ;
- un système de guidage spatial dudit robot comprenant :
• un premier repère spatial (71) fixé audit bras robotisé (62) ;
• un deuxième repère spatial (72) configuré pour être attaché en un point d'une zone d'opération ;
• un détecteur (73) configuré pour déterminer une première distance séparant ledit détecteur (73) dudit premier repère spatial (71), et une deuxième distance séparant ledit détecteur (73) dudit deuxième repère spatial (72) ;
• une unité informatique logique (52) configurée pour :
lire le quatrième fichier informatique (14) généré à l'étape (vii') de la méthode de conception,
recevoir des données concernant lesdites première et deuxième distances, et
déterminer des informations de comparaison desdites données avec les instructions d'usinage du quatrième fichier informatique (14) ;
- un outil de communication (51) connecté numériquement à ladite unité informatique pour communiquer lesdites informations de comparaison audit opérateur (D).

## Patentansprüche

1. Verfahren zum Entwerfen eines prothetischen Elements (1), umfassend die folgenden Schritte:
(i)
a) Bereitstellen einer ersten Computerdatei (11), umfassend:
- eine intraorale dreidimensionale Darstellung eines Gebisses (92), umfassend mindestens einen mithilfe des prothetischen Elements (1) wiederherzustellenden Zahn (91);
- ein radiographisches Bild des Gebisses (92);
b) Identifizieren gemeinsamer Bezugsachsen auf der intraoralen dreidimensionalen Darstellung und auf dem radiographischen Bild;
c) Vergleichen der intraoralen dreidimensionalen Darstellung mit dem radiographischen Bild, wobei dieser Teilschritt des Vergleichs eine Überlagerung der gemeinsamen Bezugsachsen umfasst;
(ii) Ermitteln einer dreidimensionalen Darstellung einer Außenseite (1A) des prothetischen Elements (1) auf Grundlage der ersten Computerdatei (11);
(iii) Ermitteln von technischen Parametern, umfassend:
- ein Zahnprotokoll, und/oder
- eine Zahnpräparationsart, und/oder
- technische Einschränkungen,
auf Grundlage der ersten Computerdatei (11), wobei mindestens einer der technischen Parameter auf Grundlage des Teilschritts des Vergleichs von Schritt (i) ermittelt wird;
(iv) Erzeugen einer zweiten Computerdatei, umfassend eine dreidimensionale Darstellung einer Volumenreduktion des mindestens einen Zahns (91) auf Grundlage der technischen Parameter;
(v) Validieren und/oder Modifizieren der zweiten Computerdatei;
(vi) Erhalten einer dreidimensionalen Darstellung einer Innenseite (1B) des prothetischen Elements (1) auf Grundlage der validierten und/oder modifizierten zweiten Computerdatei;
(vii) Erzeugen einer dritten Computerdatei (13), umfassend Informationen bezüglich der dreidimensionalen Darstellung der Außenseite (1A) und der Innenseite (1B) des prothetischen Elements (1);
(vii') Erzeugen einer vierten Computerdatei (14) auf Grundlage der validierten und/oder modifizierten zweiten Computerdatei, wobei die vierte Computerdatei (14) Anweisungen zur Bearbeitung des mindestens einen Zahns (91) entsprechend der dreidimensionalen Darstellung der Volumenreduktion des mindestens einen Zahns (91) umfasst;
(viii) Herstellen des prothetischen Elements (1) auf Grundlage der dritten Computerdatei (13).

2. Verfahren nach dem vorstehenden Anspruch, **dadurch gekennzeichnet, dass** der Schritt (ii) die folgenden Teilschritte umfasst:
(ii.1) Auswählen einer modellhaften dreidimensionalen Darstellung eines Modellgebisses in einer Datenbank auf Grundlage der ersten Computerdatei (11);
(ii.2) Auswählen eines Bereichs der modellhaften dreidimensionalen Darstellung entsprechend einem Bereich der intraoralen dreidimensionalen Darstellung entsprechend dem mindestens einen Zahn (91);
(ii.3) Validieren und/oder Modifizieren des Bereichs der modellhaften dreidimensionalen Darstellung auf Grundlage der ersten Computerdatei (11);
(ii.4) Definieren der dreidimensionalen Darstellung der Außenseite (1A) des prothetischen Elements (1) ausgehend von dem validierten und/oder modifizierten Bereich der modellhaften dreidimensionalen Darstellung.

3. Verfahren nach einem der vorstehenden Ansprüche, **dadurch gekennzeichnet, dass** der Schritt (iii) die folgenden Teilschritte umfasst:
(iii.1) Ermitteln einer Zahnpräparationsart auf Grundlage der ersten Computerdatei (11);
(iii.2) algorithmisches Erzeugen eines Zahnprotokolls auf Grundlage der im Schritt (iii.1) ermittelten Zahnpräparationsart, wobei das Zahnprotokoll aus einer Sammlung numerischer Daten besteht, um die dreidimensionalen Darstellung der Volumenreduktion geometrisch zu parametrisieren;
(iii.3) Validieren und/oder Modifizieren des Zahnprotokolls auf Grundlage des Teilschritts des Vergleichs von Schritt (i).

4. Verfahren nach dem vorstehenden Anspruch,
**dadurch gekennzeichnet, dass** der Teilschritt (iii.1) die folgenden Teilschritte umfasst:
- Visualisieren der intraoralen dreidimensionalen Darstellung eines Gebisses (92);
- Segmentieren der intraoralen dreidimensionalen Darstellung eines Gebisses (92) derart, dass eine isolierte dreidimensionale Darstellung des mindestens einen Zahns (91) erhalten wird;
- algorithmisches Erzeugen einer vestibulären, lingualen, mesialen, distalen und okklusalen Seite des mindestens einen Zahns (91) durch Identifizierung eines Punktes jeder dieser Seiten an der isolierten dreidimensionalen Darstellung des mindestens einen Zahns (91);
- Modifizieren und/oder Validieren von Grenzen der vestibulären, lingualen, mesialen, distalen und okklusalen Seite des mindestens einen Zahns (91) durch Hinzufügungen, Verlagerungen und/oder Entfernungen von Punkten dieser Seiten auf der isolierten dreidimensionalen Darstellung des mindestens einen Zahns (91);
- Identifizieren einer Markierung mindestens einer der Bezugsachsen auf Grundlage des Teilschritts des Vergleichs von Schritt (i), wobei die Markierung eine Einsatzachse des mindestens einen Zahns (91) umfasst.

5. Verfahren nach dem vorstehenden Anspruch, **dadurch gekennzeichnet, dass** die numerischen Daten des Teilschritts (iii.2) umfassen:
a) für jede Seite aus der vestibulären, lingualen, mesialen und distalen Seite des mindestens einen Zahns (91):
- einen Radius;
- eine Höhe;
entsprechend einer Parametrisierung eines querverlaufenden Abschnitts einer elliptischen Abrundung der Seite gemäß einem Ellipsenbogen einer halben Hauptachse entsprechend dem Radius, der im Wesentlichen senkrecht zur Einsatzachse gemessen wird, und einer halben Nebenachse entsprechend der Höhe, die auf im Wesentlichen parallele Weise zur Einsatzachse gemessen wird;
b) für jede Seite aus der vestibulären, lingualen, mesialen und distalen Seite des mindestens einen Zahns (91):
eine erste Verschiebungsangabe entsprechend einer Verlagerung jedes Punktes der Seite auf der isolierten dreidimensionalen Darstellung des mindestens einen Zahns zur Einsatzachse hin;
c) für die okklusale Seite des mindestens einen Zahns (91):
eine erste Verschiebungsangabe entsprechend einer Verlagerung jedes Punktes der okklusalen Seite auf der isolierten dreidimensionalen Darstellung des mindestens einen Zahns entlang der Einsatzachse;
d) zwei Prozentangaben, die zwei Bereiche definieren, die aus einem Rand- und einem mittleren Bereich des mindestens einen Zahns (91) auf der isolierten dreidimensionalen Darstellung des mindestens einen Zahns (91) bestehen, wobei jede Prozentangabe dem Verhältnis zwischen einer Höhe eines Bereichs und einer Höhe der zwei Bereiche entspricht, wobei diese Höhen im Wesentlichen parallel zur Einsatzachse gemessen werden;
e) für jeden der Bereiche:
- einen Ausrichtungswinkel der Volumenreduktion, der in Bezug auf die Einsatzachse gemessen wird;
- eine Mindesthöhe.

6. Verfahren nach dem vorstehenden Anspruch, **dadurch gekennzeichnet, dass** die technischen Einschränkungen eine Mindestdicke eines Entwurfsmaterials des prothetischen Elements (1) umfassen und dadurch, dass die ersten Verschiebungsangaben der Seiten von dieser Mindestdicke abhängen.

7. Verfahren nach einer der zwei vorstehenden Ansprüche, **dadurch gekennzeichnet, dass** der Schritt (iv) einen Teilschritt des algorithmischen Erzeugens einer dreidimensionalen Darstellung einer Volumenreduktion des mindestens einen Zahns (91) umfasst, wobei dieser Teilschritt die folgenden Teilschritte umfasst:
(iv.1) Erzeugen einer Randoberfläche, die aus den elliptischen Abrundungen (201) der vestibulären, lingualen, mesialen und distalen Seite des mindestens einen Zahns (91) besteht;
(iv.2) Erzeugen einer konischen ersten Oberfläche (202) um die Einsatzachse (Z) herum ausgehend von einer äußersten Wölbung, die an die im Teilschritt (iv.1) erzeugte Randoberfläche angrenzt, wobei diese konische erste Oberfläche sich parallel zum Randbereich erstreckt und eine Neigung zur Einsatzachse hin bei einem Winkel entsprechend dem Ausrichtungswinkel der Volumenreduktion des Randbereichs aufweist;
(iv.3) Erzeugen einer konischen zweiten Oberfläche (203) um die Einsatzachse herum ausgehend von einer äußersten Wölbung, die an die im Teilschritt (iv.2) erzeugte konische erste Oberfläche angrenzt, wobei diese konische zweite Oberfläche sich parallel zum mittleren Bereich erstreckt und eine Neigung zur Einsatzachse hin bei einem Winkel entsprechend dem Ausrichtungswinkel der Volumenreduktion des mittleren Bereichs aufweist;
(iv.4)
• Berechnen einer zweiten Verschiebungsangabe für jeden Punkt des Rand- und des mittleren Bereichs auf der isolierten dreidimensionalen Darstellung des mindestens einen Zahns, wobei diese zweite Verschiebungsangabe einer Verlagerung dieses Punktes zur Einsatzachse hin oder in Gegenrichtung zu dieser entspricht, um diesen Punkt auf einer der konischen ersten oder zweiten Oberfläche zu verlagern;
• Definieren einer primären Reduktionsoberfläche durch Verlagern jedes Punktes des Rand- und des mittleren Bereichs zur Einsatzachse hin gemäß:
- der zweiten Verschiebungsangabe dieses Punktes, wenn sie einer Verlagerung zur Einsatzachse hin entspricht und wenn sie größer ist als die erste Verschiebungsangabe dieses Punktes,
- andernfalls der ersten Verschiebungsangabe dieses Punktes;
(iv.5) Erzeugen einer okklusalen Oberfläche (204) ausgehend von einer äußersten Wölbung, die an die im Teilschritt (iv.4) erzeugte primäre Reduktionsoberfläche angrenzt, durch eine Verlagerung jedes Punktes der okklusalen Seite auf der isolierten dreidimensionalen Darstellung des mindestens einen Zahns entlang der Einsatzachse gemäß der ersten Verschiebungsangabe;
(iv.6) Glätten und/oder Ausgleichen einer Gesamtoberfläche (205), die aus der Zusammenstellung der Randoberflächen, der primären Reduktions- und okklusalen Oberfläche besteht, wobei diese Gesamtoberfläche in einem Schnitt mit der Einsatzachse senkrecht zur Einsatzachse eine zur Gesamtoberfläche tangentiale Ebene aufweist.

8. Verfahren nach einem der vorstehenden Ansprüche, **dadurch gekennzeichnet, dass**:
- die zweite Computerdatei aus einer modifizierbaren Datei im STL-Format besteht;
- der Schritt (v) eine Validierung und/oder eine Modifizierung jedes aus geometrischen Parametern bezüglich der dreidimensionalen Darstellung der Volumenreduktion des mindestens einen Zahns (91) in einer Gesamtheit von zulässigen Werten umfasst, die durch mindestens einen der im Schritt (iii) ermittelten technischen Parameter vorab definiert wird.

9. Entwurfsverfahren nach einem der vorstehenden Ansprüche, **dadurch gekennzeichnet, dass**:
- die Informationen der dritten Computerdatei (13) Anweisungen zur Bearbeitung eines Materials umfassen;
- der Schritt (viii) einen Teilschritt der Bearbeitung des Materials auf Grundlage der Bearbeitungsanweisungen umfasst.

10. Entwurfsverfahren nach einem der vorstehenden Ansprüche, weiter die folgenden zusätzlichen Schritte umfassend:
(vii") Erzeugen einer fünften Computerdatei auf Grundlage der zweiten validierten und/oder modifizierten Computerdatei, wobei die fünfte Computerdatei Informationen bezüglich der im Schritt (vi) erhaltenen dreidimensionalen Darstellung der Innenseite (1B) des prothetischen Elements (1) umfasst, wobei diese Informationen Anweisungen zur Bearbeitung eines steifen Ausgangsmaterials entsprechend der dreidimensionalen Darstellung der Innenseite (1B) des prothetischen Elements (1) umfassen;
(viii") Bearbeiten eines Blocks des steifen Ausgangsmaterials auf Grundlage der Bearbeitungsanweisungen der fünften Computerdatei (13) derart, dass ein Steuerungsschlüssel (100) einer Bearbeitung des mindestens einen Zahns (91) entsprechend einer Bearbeitung gemäß der dreidimensionalen Darstellung der Volumenreduktion des mindestens einen Zahns (91) produziert wird;
und weiter umfassend einen zusätzlichen Schritt des Schneidens des Steuerungsschlüssels (100) in Scheiben (101) parallel zu einer Ebene.

11. Entwurfsverfahren nach Anspruch 7, weiter die folgenden zusätzlichen Schritte umfassend:
(iv') Erzeugen einer sechsten Computerdatei auf Grundlage der ersten Computerdatei (11) und des im Schritt (iii.3) validierten und/oder modifizierten Zahnprotokolls, wobei die sechste Computerdatei drei Sammlungen von Anweisungen zur Bearbeitung eines steifen Ausgangsmaterials umfasst, wobei jede dieser Sammlungen Bearbeitungsanweisungen umfasst, um einen Hohlraum (91') im steifen Ausgangsmaterial entsprechend der isolierten dreidimensionalen Darstellung zu schaffen,
wobei eine erste der Sammlungen von Anweisungen weiter Bearbeitungsanweisungen umfasst, um ein mindestens teilweise konisches Zugangsfenster (111) um den Hohlraum (91') herum, der konischen ersten und zweiten Oberfläche folgend, zu schaffen,
wobei eine zweite der Sammlungen weiter Bearbeitungsanweisungen umfasst, um zwei Fenster (121) für einen oberen Zugang zum Hohlraum (9T) zu schaffen, der an die mesiale und die distale Oberfläche des Hohlraums angrenzt, die der mesialen und der distalen Seite des mindestens einen Zahns (91) auf der isolierten dreidimensionalen Darstellung entsprechen,
wobei eine dritte der Sammlungen weiter Anweisungen zur Bearbeitung eines Teils des steifen Ausgangsmaterials umfasst, das einen mittleren Bereich des Hohlraums entsprechend dem mittleren Bereich des mindestens einen Zahns (91) auf der isolierten dreidimensionalen Darstellung umgibt, um zwei abfallende Kanten (131) gemäß dem Ausrichtungswinkel der Volumenreduktion des mittleren Bereichs zu schaffen;
(viiï') Bearbeiten eines ersten, eines zweiten und eines dritten Blocks des steifen Ausgangsmaterials auf Grundlage der ersten, zweiten bzw. dritten Sammlung von Bearbeitungsanweisungen der sechsten Computerdatei derart, dass drei Führungen (110, 120, 130) zur Bearbeitung des mindestens einen Zahns (91) gemäß der dreidimensionalen Darstellung der Volumenreduktion des mindestens einen Zahns (91) produziert werden.

12. Entwurfsverfahren nach dem vorstehenden Anspruch, **dadurch gekennzeichnet, dass** es weiter den zusätzlichen Schritt des Anbringens mindestens eines Sicherheitsanschlags und einer Schiene zur Positionierung eines Zahnbohrers auf den Führungen (110, 120, 130) umfasst.

13. Gesamtheit von Einrichtungen zum Entwerfen eines prothetischen Elements (1) durch Ausführung des Entwurfsverfahrens nach einem der vorstehenden Ansprüche, wobei die Gesamtheit von Einrichtungen umfasst:
a) mindestens eine Bildgebungseinrichtung (2), um die erste Computerdatei des Schritts (i) des Entwurfsverfahrens bereitzustellen;
b) Mittel zum Ausführen der Schritte der Identifizierung und der Überlagerung gemeinsamer Bezugsachsen von Schritt (i) und
c) ein Computersystem (3), um die Schritte (ii) bis (vii) und (vii') des Entwurfsverfahrens auszuführen, umfassend:
- eine Schnittstelle zum Empfangen:
mindestens eines technischen Parameters, der im Schritt (iii) des Entwurfsverfahrens ermittelt wird, und
von Validierungen und/oder von Modifizierungen der zweiten Computerdatei des Schritts (v) des Entwurfsverfahrens;
und zum Visualisieren und/oder Kommunizieren von Angaben über:
die intraorale dreidimensionale Darstellung und das radiographische Bild der ersten Computerdatei (11), die im Schritt (i) bereitgestellt wird;
die im Schritt (ii) erhaltene dreidimensionale Darstellung der Außenseite (A1) des prothetischen Elements (1);
die dreidimensionale Darstellung der Volumenreduktion des mindestens einen Zahns (91) der im Schritt (iv) erzeugten zweiten Computerdatei;
die dreidimensionale Darstellung der Volumenreduktion des mindestens einen Zahns (91) der im Schritt (v) validierten und/oder modifizierten zweiten Computerdatei;
die im Schritt (vi) erhaltene dreidimensionale Darstellung der Innenseite (1B) des prothetischen Elements (1);
- eine Logikeinheit, um die Schritte (ii), (iv), (vii) und (vii') des Entwurfsverfahrens durchzuführen;
d) eine Produktionsmaschine (4), um die Informationen der im Schritt (vii) erzeugten dritten Computerdatei (13) zu lesen und um den Schritt (viii) des Entwurfsverfahrens durchzuführen.

14. Gesamtheit von Einrichtungen nach dem vorstehenden Anspruch, wobei das Informatiksystem (3) einen Computer umfasst und wobei die Logikeinheit definiert ist durch:
a) einen Prozessor des Computers und
b) mindestens ein computerlesbares Medium,
auf dem gespeichert sind:
- ein erstes Computerprogramm, umfassend erste Anweisungen, die, wenn das erste Computerprogramm ausgeführt wird, die Durchführung des Schritts (iv) des Entwurfsverfahrens nach einem der Ansprüche 1 bis 12 bewirken;
- ein zweites Computerprogramm, umfassend zweite Anweisungen, die, wenn das zweite Computerprogramm ausgeführt wird, die Durchführung des Schritts (vii) des Entwurfsverfahrens nach einem der Ansprüche 1 bis 12 bewirken; und
- ein drittes Computerprogramm, umfassend dritte Anweisungen, die, wenn das dritte Computerprogramm ausgeführt wird, die Durchführung des Schritts (vii') des Entwurfsverfahrens nach einem der Ansprüche 1 bis 12 bewirken.

15. Kit, umfassend eine Gesamtheit von Einrichtungen nach Anspruch 13 oder 14, und ein Hilfssystem (5) für einen Operator (D) bei einer Zahnwiederherstellung, umfassend:
a) einen Roboter (6), der mit einem Roboterarm (61) ausgestattet ist, der beweglich ist und an einem Bearbeitungswerkzeug (62) angebracht ist, das an einem Ende des Roboterarms (61) platziert ist;
b) ein Raumführungssystem des Roboters, umfassend:
- eine erste Raummarkierung (71), die am Roboterarm (62) fixiert ist;
- eine zweite Raummarkierung (72), die dazu ausgelegt ist, an einem Punkt eines Operationsbereichs befestigt zu werden;
- einen Sensor (73), der dazu ausgelegt ist, einen ersten Abstand, der den Sensor (73) von der ersten Raummarkierung (71) trennt, und einen zweiten Abstand, der den Sensor (73) von der zweiten Raummarkierung (72) trennt, zu ermitteln;
- eine Logikinformatikeinheit (52), die ausgelegt ist zum:
Lesen der im Schritt (vii') erzeugten vierten Computerdatei (14) des Entwurfsverfahrens,
Empfangen von Angaben hinsichtlich des ersten und des zweiten Abstandes, und
Ermitteln von Informationen des Vergleichs der Angaben mit den Bearbeitungsanweisungen der vierten Computerdatei (14);
c) ein Kommunikationswerkzeug (51), das numerisch mit der Informatikeinheit verbunden ist, um dem Operator (D) die Vergleichsinformationen mitzuteilen.

## Claims

1. A method for designing a prosthetic element (1) comprising the following steps:
(i)
● providing a first computer file (11) comprising:
- an intra-oral three-dimensional representation of a dentition (92) comprising at least one tooth (91) to be restored by means of said prosthetic element (1);
- a radiographic image of said dentition (92);
● identifying common reference axes on said intra-oral three-dimensional representation and on said radiographic image;
● comparing said intra-oral three-dimensional representation with said radiographic image, this comparison sub-step comprising an overlay of said common reference axes;
(ii) determining a three-dimensional representation of an extrados (1A) of said prosthetic element (1) on the basis of said first computer file (11);
(iii) determining technical parameters comprising:
- a dental protocol, and/or
- a type of dental preparation, and/or
- technical constraints,
on the basis of said first computer file (11), at least one of said technical parameters being determined on the basis of the comparison sub-step of the step (i);
(iv) generating a second computer file comprising a three-dimensional representation of a volumetric reduction of said at least one tooth (91) on the basis of said technical parameters;
(v) validating and/or modifying said second computer file;
(vi) obtaining a three-dimensional representation of an intrados (1B) of said prosthetic element (1) on the basis of said second validated and/or modified computer file;
(vii) generating a third computer file (13) comprising information relating to the three-dimensional representations of said extrados (1A) and intrados (1B) of said prosthetic element (1);
(vii') generating a fourth computer file (14) on the basis of said second validated and/or modified computer file, said fourth computer file (14) comprising machining instructions for said at least one tooth (91) corresponding to said three-dimensional representation of said volumetric reduction of said at least one tooth (91);
(viii) producing said prosthetic element (1) on the basis of said third computer file (13).

2. The method according to the preceding claim, **characterised in that** the step (ii) comprises the following sub-steps:
(ii.1) selecting a three-dimensional representation model of a model dentition from a database on the basis of said first computer file (11);
(ii.2) selecting a zone of the three-dimensional representation model corresponding to a zone of the intra-oral three-dimensional representation corresponding to said at least one tooth (91);
(ii.3) validating and/or modifying the zone of the three-dimensional representation model on the basis of said first computer file (11);
(ii.4) defining the three-dimensional representation of the extrados (1A) of said prosthetic element (1) from the validated and/or modified zone of the three-dimensional representation model.

3. The method according to any of the preceding claims **characterised in that** the step (iii) comprises the following sub-steps:
(iii.1) determining a type of dental preparation on the basis of said first computer file (11);
(iii.2) algorithmically generating a dental protocol on the basis of the type of dental preparation determined in the step (iii.1),
said dental protocol consisting of a collection of numerical data to geometrically parameterise said three-dimensional representation of the volumetric reduction;
(iii.3) validating and/or modifying said dental protocol on the basis of the comparison sub-step of the step (i).

4. The method according to the preceding claim,
**characterised in that** the sub-step (iii.1) comprises the following sub-steps:
● visualizing said intra-oral three-dimensional representation of a dentition (92);
● segmenting said intra-oral three-dimensional representation of a dentition (92) so as to obtain an isolated three-dimensional representation of the at least one tooth (91);
● algorithmically generating vestibular, lingual, mesial, distal and occlusal faces of the at least one tooth (91) by identifying a point on each of these faces at the isolated three-dimensional representation of the at least one tooth (91);
● modifying and/or validating boundaries of said vestibular, lingual, mesial, distal and occlusal faces of the at least one tooth (91) by adding, moving and/or removing points of these faces on the isolated three-dimensional representation of the at least one tooth (91);
● identifying a reference frame of at least one of said reference axes on the basis of the comparison sub-step of the step (i), the reference frame comprising an insertion axis of the at least one tooth (91).

5. The method according to the preceding claim, **characterised in that** the numerical data of sub-step (iii.2) comprise:
- for each face among the vestibular, lingual, mesial and distal faces of said at least one tooth (91):
● a radius;
● a height;
corresponding to a parameterisation of a transverse section of an elliptical fillet of the face along an elliptical arc of semi-major axis corresponding to said radius measured essentially perpendicularly to said insertion axis and of semi-minor axis corresponding to said height measured essentially parallel to said insertion axis;
- for each of the vestibular, lingual, mesial and distal faces of said at least one tooth (91):
a first offset data corresponding to a displacement of each point of the face on the isolated three-dimensional representation of the at least one tooth towards the insertion axis;
- for the occlusal face of said at least one tooth (91):
a first offset data corresponding to a displacement of each point of the occlusal face on the isolated three-dimensional representation of the at least one tooth along the insertion axis;
- two percentages defining two zones consisting of marginal and middle zones of the at least one tooth (91) on the isolated three-dimensional representation of the at least one tooth (91), each percentage corresponding to the ratio between a height of one zone and a height of both zones, these heights being measured essentially parallel to the insertion axis;
- for each of said zones:
● a volumetric reduction orientation angle measured with respect to the insertion axis;
● a minimum height.

6. The method according to the preceding claim, **characterised in that** the technical constraints comprise a minimum thickness of a material for the design of the prosthetic element (1), and **in that** the first offset data of the faces depend on this minimum thickness.

7. The method according to either of the two preceding claims, **characterised in that** the step (iv) comprises a sub-step of algorithmic generation of a three-dimensional representation of a volumetric reduction of said at least one tooth (91), this sub-step comprising the following sub-steps:
(iv.1) generating a marginal surface consisting of the elliptical fillets (201) of the vestibular, lingual, mesial and distal faces of said at least one tooth (91);
(iv.2) generating a first conical surface (202) around said insertion axis (Z) from an end curve bordering the marginal surface generated in the sub-step (iv.1), this first conical surface extending parallel to said marginal zone and having an inclination towards the insertion axis of an angle corresponding to the volumetric reduction orientation angle of said marginal zone;
(iv.3) generating a second conical surface (203) around said insertion axis from an end curve bordering the first conical surface generated in the sub-step (iv.2), this second conical surface extending parallel to said middle zone and having an inclination towards the insertion axis of an angle corresponding to the orientation angle of volumetric reduction of said middle zone;
(iv.4)
● calculating a second offset data for each point of the marginal and middle zones on the isolated three-dimensional representation of the at least one tooth, this second offset data corresponding to a displacement of this point towards or away from the insertion axis to displace this point on one of the first or second conical surfaces;
● defining a primary reduction surface by moving each point from the marginal and middle zones towards the insertion axis according to:
- the second offset data of this point if it corresponds to a displacement towards the insertion axis and if it is larger than the first offset data of this point,
- the first offset data of this point otherwise;
(iv.5) generating an occlusal surface (204) from an end curve bordering the primary reduction surface generated in the sub-step (iv.4) by a displacement of each point on the occlusal face on the isolated three-dimensional representation of the at least one tooth along the insertion axis according to the first offset data;
(iv.6) smoothing and/or regularising a total surface (205) consisting of the assembly of the marginal, primary reduction and occlusal surfaces, this total surface having a plane tangent to the total surface at an intersection with the insertion axis perpendicular to the insertion axis.

8. The method according to any of the preceding claims, **characterised in that**:
- said second computer file consists of a file of modifiable STL format;
- the step (v) comprises a validation and/or a modification of each of the geometric parameters relating to said three-dimensional representation of the volumetric reduction of said at least one tooth (91) in a set of admissible values previously defined by at least one of said technical parameters determined in the step (iii).

9. The design method according to any of the preceding claims **characterised in that**:
- said information of said third computer file (13) comprises instructions for machining a material;
- the step (viii) comprises a sub-step of machining said material on the basis of said machining instructions.

10. The design method according to any of the preceding claims, further comprising the following additional steps:
(vii") generating a fifth computer file on the basis of said second validated and/or modified computer file, said fifth computer file comprising information relating to the three-dimensional representation of the intrados (1B) of said prosthetic element (1) obtained in the step (vi), this information comprising instructions for machining a rigid raw material corresponding to said three-dimensional representation of the intrados (1B) of said prosthetic element (1);
(viii") machining a block of said rigid raw material on the basis of the machining instructions of said fifth computer file (13), so as to produce a control key (100) of a machining of said at least one tooth (91) corresponding to a machining according to said three-dimensional representation of the volumetric reduction of said at least one tooth (91);
and further comprising the additional step of cutting the control key (100) into slices (101) parallelly to a plane.

11. The design method according to claim 7, further comprising the following additional steps:
(iv') generating a sixth computer file on the basis of said first computer file (11) and said dental protocol validated and/or modified in the step (iii.3), the sixth computer file comprising three collections of instructions for machining a rigid raw material, each of these collections comprising machining instructions for creating a cavity (91') in the rigid raw material corresponding to the isolated three-dimensional representation,
a first of the collections of instructions further comprising machining instructions for creating an access window (111) at least partially conical around the cavity (91') following the first and second conical surfaces,
a second of the collections of instructions further comprising machining instructions for creating two windows (121) of upper access to the cavity (91') bordering mesial and distal faces of the cavity which correspond to the mesial and distal faces of said at least one tooth (91) on the isolated three-dimensional representation,
a third of the collections of instructions further comprising instructions for machining a portion of the rigid raw material surrounding a middle zone of the cavity corresponding to the middle zone of said at least one tooth (91) on the isolated three-dimensional representation, to create two sloping edges (131) according to the orientation angle of volumetric reduction of the middle zone;
(viii') machining a first, a second and a third block of said rigid raw material respectively on the basis of the first, second and third collections of machining instructions of said sixth computer file, so as to produce three guides (110, 120, 130) for machining the at least one tooth (91) according to said three-dimensional representation of the volumetric reduction of the at least one tooth (91).

12. The design method according to the preceding claim, **characterised in that** it further comprises the additional step of interlocking at least one safety abutment and a rail for positioning a dental drill on the guides (110, 120, 130).

13. A set of apparatus for designing a prosthetic element (1) by carrying out the design method according to any of the preceding claims, the set of apparatus comprising:
- at least one imaging apparatus (2) for providing the first computer file of the step (i) of the design method;
- means for carrying out the steps of identifying and overlaying of the common reference axes of step (i) and
- a computer system (3) for implementing steps (ii) to (vii) and (vii') of the design method, comprising:
● an interface for receiving:
at least one technical parameter determined in the step (iii) of the design method, and
validations and/or modifications of the second computer file of the step (v) of the design method;
and to visualise and/or communicate data on:
the intra-oral three-dimensional representation and the radiographic image of the first computer file (11) provided in the step (i);
the three-dimensional representation of the extrados (1A) of said prosthetic element (1) obtained in the step (ii);
the three-dimensional representation of the volumetric reduction of the at least one tooth (91) of the second computer file generated in the step (iv);
the three-dimensional representation of the volumetric reduction of the at least one tooth (91) of the second computer file validated and/or modified in the step (v);
the three-dimensional representation of the intrados (1B) of said prosthetic element (1) obtained in the step (vi);
● a logic unit for implementing the steps (ii), (iv), (vi), (vii) and (vii') of the design method;
- a production machine (4) for reading the information from the third computer file (13) generated in the step (vii), and for implementing the step (viii) of the design method.

14. The set of apparatus according to the preceding claim,
wherein the computer system (3) comprises a computer and wherein the logic unit is defined by:
- a processor of the computer and
- at least one computer-readable medium on which is recorded:
∘ a first computer program comprising first instructions which, when said first computer program is executed, lead to the implementation of the step (iv) of the design method according to any one of claims 1 to 12;
∘ a second computer program comprising second instructions which, when said second computer program is executed, lead to the implementation of the step (vii) of the design method according to any of claims 1 to 12; and
∘ a third computer program comprising third instructions which, when said third computer program is executed, lead to the implementation of the step (vii') of the design method according to any of claims 1 to 12.

15. A kit comprising a set of apparatus according to claim 13 or 14, and a system for assisting (5) an operator (D) in a dental restoration comprising:
- a robot (6) equipped with a mobile robotic arm (61) and attached to a machining tool (62) placed at one end of said robotic arm (61);
- a spatial guidance system for said robot comprising :
● a first spatial reference frame (71) fixed to said robot arm (62);
● a second spatial reference frame (72) configured to be attached to one point in an operating zone;
● a detector (73) configured to determine a first distance between said detector (73) and said first spatial reference frame (71), and a second distance between said detector (73) and said second spatial reference frame (72);
● a computer logic unit (52) configured to:
read the fourth computer file (14) generated in step (vii') of the design method,
receive data on said first and second distances, and
determine information for comparing said data with the machining instructions of the fourth computer file (14);
- a communication tool (51) digitally connected to said computer unit to communicate said comparison information to said operator (D).
